# EUROPÄISCHE PATENTSCHRIFT

(11) **EP 1 097 132 B1**
(45) Veröffentlichungstag und Bekanntmachung des Hinweises auf die Patenterteilung: **17.12.2003**
(21) Anmeldenummer: 99932730.7
(22) Anmeldetag: 25.06.1999
(51) Int. Cl.: C07C 401/00

(54) **FUNKTIONELLE VITAMIN-D-DERIVATE UND VERFAHREN ZUR BESTIMMUNG VON 25-HYDROXY- UND 1ALPHA,25-DIHYDROXY-VITAMIN-D**
FUNCTIONAL VITAMIN D DERIVATIVES AND A METHOD FOR DETERMINING 25-HYDROXY-VITAMIN D AND 1ALPHA,25-DIHYDROXY-VITAMIN D
DERIVES FONCTIONNELS DE LA VITAMINE D ET PROCEDE POUR LA DETERMINATION DE 25-HYDROXY- ET 1ALPHA,25-DIHYDROXY-VITAMINE D

(30) Priorität: 25.06.1998 DE 19828379; 04.09.1998 DE 19840435
(43) Veröffentlichungstag der Anmeldung: 09.05.2001
(73) Patentinhaber: Immundiagnostik Gesellschaft für Produktion und Vertrieb von Labordiagnostika mbH, 64625 Bensheim (DE); Biomedica GmbH, 1210 Wien (AT)
(72) Erfinder: ARMBRUSTER, Franz, Paul, D-67240 Bobenheim-Roxheim (DE); VOELTER, Wolfgang, D-72070 Tübingen (DE); SCHWING, Jens, D-55128 Mainz (DE); BIRKMAYER, Christian, D-80538 München (DE)
(74) Vertreter: Benedum, Ulrich Max, Dr.
(86) Internationale Anmeldenummer: EP9904418
(87) Internationale Veröffentlichungsnummer: WO99067211

(56) Entgegenhaltungen:
- EP-A- 0 312 360
- WO-A-97/24127
- KOBAYASHI, N. ET AL.: "Specificity of Polyclonal Antibodies Raised against a Novel 24,25-Dihydroxyvitamin D3-Bovine Serum Albumin Conjugant Linked through the C-11-alpha or C-3 Position" J. STEROID BIOCHEM. MOLEC. BIOL., Bd. 62, Nr. 1, 1997, Seiten 79-87, XP002119849
- HIGASHI, T. ET AL.: "Enzyme-linked immunosorbent assay for plasma 24,25-dihydroxyvitamin-D3" ANALYTICA CHIMICA ACTA, Bd. 365, Nr. 1-3, 1998, Seiten 151-158, XP002119850

## Beschreibung

Die Erfindung betrifft Verfahren zur Herstellung eines 25-Hydroxyvitamin-D-Derivats, deren Herstellung sowie Verfahren zur Bestimmung von 25-Hydroxy- und 1,25-Dihydroxy-Vitamin-D in Proben.

Die D-Vitamine oder Calciferole entstehen aus ihren Provitaminen durch eine Sonnenlichtkatalysierte Spaltung des B-Rings im Sterangerüst. Ihre wichtigsten Vertreter sind Vitamin-D₃ (Cholecalciferol) und Vitamin-D₂ (Ergocalciferol), welche sich nur in den Seitenketten geringfügig unterscheiden, die aber - soweit bekannt - gleich metabolisiert werden und identische biologische Wirkungen besitzen. Während Provitamin-D₂ mit der Nahrung aufgenommen werden muss, kann das Provitamin-D₃ im menschlichen Organismus gebildet werden. Soweit durch Indizes nicht näher bezeichnet, umfasst die Bezeichnung Vitamin-D nachstehend allgemein alle Vitamin-D-Formen. In der Haut gebildetes oder mit der Nahrung aufgenommenes Vitamin-D wird im Plasma vom Vitamin-D-Bindungs- oder -Transportprotein (DBP) gebunden, zur Leber transportiert und dort zu 25-Hydroxyvitamin-D (25-OH-D) metabolisiert. Das Vitamin-D-bindende Protein DBP ist auch als Gc-Globulin oder *group specific* component bekannt (J.G. Haddad in J. Steroid Biochem. Molec. Biol. (1995) 53, 579-582). Über 95% des im Serum messbaren 25-Hydroxyvitamin-D ist in der Regel 25-Hydroxyvitamin-D₃- 25-Hydroxyvitamin-D₂ wird nur in höheren Anteilen gefunden, wenn die Person unter einer Medikation mit Vitamin-D₂ steht oder, wie in den Vereinigten Staaten häufig praktiziert, die Nahrung mit Vitamin-D₂ angereichert wird.

25-Hydroxyvitamin-D ist der vorherrschende Vitamin-D-Metabolit im Blutkreislauf und seine Konzentration im Serum beschreibt allgemein den Vitamin-D-Status, d.h., inwieweit dem Organismus Vitamin-D zur Verfügung steht. 25-Hydroxyvitamin-D wird bei Bedarf in der Niere zu 1α,25-Dihydroxyvitamin-D metabolisiert, einer hormonartigen Substanz mit hoher biologischer Wirkung. Die Bestimmung von 1α,25-Dihydroxyvitamin-D gibt an, wieviel Vitamin-D sich in der aktivierten Form befinden.

Die Bestimmung von 25-Hydroxyvitamin-D in einer Probe erfolgt bevorzugt nach dem Prinzip der kompetitive Proteinbindungsanalyse, wobei anhand der Verdrängung von radioaktivem 25-Hydroxyvitamin-D von den Bindungsstellen eines Vitamin-D-bindenden Proteins das in der Probe vorhandene 25-Hydroxyvitamin-D quantifiziert wird. Daneben haben sich seit einigen Jahren Radioimmunoassays mit ¹²⁵I-markierten Vitamin-D-Derivaten und Antikörpern gegen Vitamin-D-Derivate in der Diagnostik etabliert. Die Angaben zum üblichen 25-Hydroxyvitamin-D-Spiegel im Serum schwanken je nach Labor. Es besteht aber Einigkeit dahin, dass die Konzentration von 25-Hydroxyvitamin-D im Serum in der Regel größer als 5 ng/ml und kleiner als 80 ng/ml ist. Die kompetitive Proteinbindungsanalyse verlangt den Einsatz eines radioaktiven Vitamin-D-Derivats, welches das gleiche Proteinbindungsverhalten besitzen muss wie 25-Hydroxyvitamin-D. Ähnliches gilt auch für die kompetitive Bindungsanalyse für das biologisch aktive 1α,25-Dihydroxyvitamin-D oder andere Vitamin-D-Metaboliten.

Die europäischen Patentschriften 0 312 360 und 0 363 211 sowie Tanabe et al. in J. Chem. Soc., Chem. Commun. 1989, 1220-1221 und J. Nutri. Sci. Vitaminol., 1991, 37, 139-147 offenbaren verschiedene ¹²⁵Iod-markierte Hydroxy- und Dihydroxyvitamin-D-Derivate und deren Verwendung in Bindungsanalysen. Nachteilig an diesen Derivaten ist, dass sie problematisch herzustellen und extrem labil sind. Licht, radioaktive Strahlung, Protonen, Wasserstoff Enzyme, freie Radikale oder die Gegenwart von Iod in freier oder gebundener Form haben großen Einfluss auf die Raumstruktur und die Bindeeigenschaften der Vitamin-D-Derivate an Vitamin-D-bindendes Protein DBP oder spezifischen Antikörpern. Sie können vor allem eine Drehung des A-Rings im Sterangerüst verursachen oder katalysieren. Die 3β-Hydroxygruppe des Vitamin-D-Moleküls wird dabei in die Pseudo-1α-Position gedreht, so dass dann das 5,6-trans-Vitamin-D vorliegt. Die sogenannten Pseudo-1α-Hydroxy-Analogen des Vitamin-D werden zwar ähnlich wie Vitamin-D metabolisiert, sie haben aber eine in wesentlichen Punkten andere Struktur und werden von Vitamin-D-bindenden Proteinen wie bspw. DBP/Gc-Globulin oder Anti-Vitamin-D-Antikörper nicht oder deutlich schlechter gebunden. 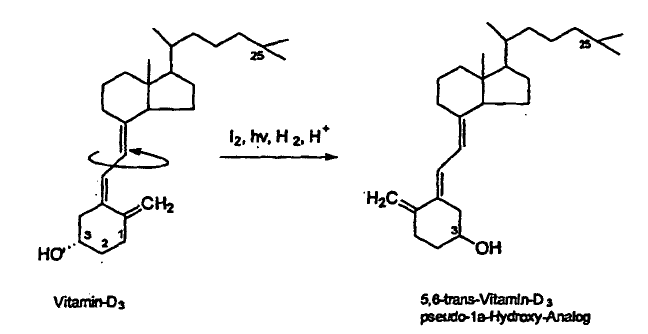 Die vorstehend beschriebene Umlagerung ist als Beispiel zu verstehen. Es treten auch andere chemische Reaktionen und Umlagerungen auf. Gleiches gilt für ³H- oder ¹⁴C-markierte Vitamin-D-Derivate. Auch diese Vitamin-D-Derivate sind nicht so stabil, dass sie eine zuverlässige Bindungsanalyse erlauben. Die radioaktive Markierung steigert zudem die Kosten für Lagerung, Transport und Entsorgung und ist allgemein nachteilig für Gesundheit und Umwelt. Ferner ist die Halbwertszeit von ¹²⁵Iod vergleichsweise kurz. Eine kompetitive Bindungsanalyse mit ³H- und ¹⁴Cmarkierten Vitamin-D-Derivaten hingegen verlangt besondere Szintillationszähler und ist apparativ anspruchsvoller, bei weitgehend gleichen Problemen.

Ray et al. in Biochemistry, 1991, 30, 4809-4813 offenbaren die Kopplung von Vitamin-D₃ mit verschiedenen farbgebenden Gruppen. Die Nachweisempfindlichkeit für die farbstoffmarkierten Vitamin-D₃-Derivate ist aber zu gering, als dass man sie in einer kompetitiven Bindungsanalyse für natürliche Vitamin-D-Metabolite einsetzen könnte, abgesehen davon, dass die farbstoffmarkierten Derivate in Serum nicht stabil und zudem besonders lichtempfindlich sind.

Die Kopplung von Hydroxyvitamin-D mit den farbgebenden Gruppen erfolgt im ersten Schritt über eine Cyanoethylierung von 25-Hydroxyvitamin-D (a.a.O. Seite 4810, rechte Spalte). Das cyanoethylierte Hydroxyvitamin-D wird dann mit einem Gemisch von Lithiumaluminiumhydrid und Aluminiumtriclorid zum entsprechenden Amin reduziert. Die reaktive Aminogruppe dient dann als Kopplungsgruppe für farbstoffmarkierte Vitamin-D-Verbindung. Die Amin-Zwischenverbindung wird dabei ohne vorherige Analyse eingesetzt. Die resultierenden Farbstoffgekoppelten Vitamin-D-Derivate können, werden sie in einem vielfachen Konzentrationsüberschuss eingesetzt, 25-Hydroxyvitamin-D aus dem dem Vitamin-D-Bindungsprotein verdrängen.

Ferner wird von Roy et al. in Steroids, 1995, 60, 530-533 die Aminopropylierung von 1α,25-Dihydroxyvitamin-D beschrieben und eine Kopplung des Reaktionsprodukts an einen makromolekularen Träger für Affinitätsstudien. Die Amin-Zwischenverbindung wird ohne nähere Analyse in die Kopplungsreaktion eingesetzt.

WO-A-97/24127 (Ray et al.) beschreibt eine Vielzahl von verschiedenen funktionelle Vitamin-D-Derivaten, die in gewissen Umfang 25-Hydroxyvitamin-D am Vitamin-D-bindenden Protein (DBP) verdrängen können. Es wird zwar die Synthese von funktionellen 25-Hydroxyvitamin-D-Derivaten skizziert, aber nicht angegebenen, wie diese Verbindungen tatsächlich erhalten werden können.

Vitamin-D-Derivaten, die in einer kompetitiven Bindungsanalyse oder ganz generell in Immunoassays von Vitamin-D-Metaboliten wie 25-Hydroxy- und 1,25-Dihydroxy-Vitamin-D eingesetzt werden können. Dies setzt folgende Eigenschaften voraus, erstens, dass für die Vitamin-D-Derivate eine Nachweisempfindlichkeit besteht, welche höher ist, beziehungsweise in einem niedrigeren Konzentrationsbereich liegt, als die Konzentration der gesuchten Vitamin-D-Metabolite in den Proben; zweitens, dass die Derivate in Serum, Plasma oder Urin unter üblichen protischen Bedingungen und gegenüber Serumenzymen stabil sind; und schließlich drittens, dass die Derivate über Wochen und Monate hinreichend licht- und lagerstabil sind. Die hierin beschriebenen Vitamin-D-Derivate haben die Formel 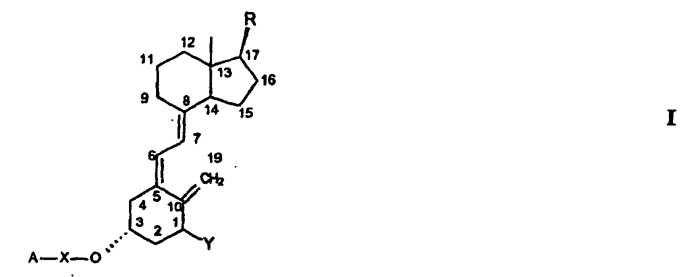 worin ist:
- O: das Sauerstoffatom einer Ethergruppe;
- X: eine substituierte oder unsubstituierte Kohlenwasserstoffstoffgruppe von 0,8 bis 4,2 nm Länge, bevorzugt ein C8- bis C12-Gruppe, die übliche Heteroatome wie S, O, N oder P aufweisen kann, ganz besonders bevorzugt eine Hexamido-, Octamido- oder Decamido-Amidopropylether-Linkergruppe;
- Y: Wasserstoff oder eine Hydroxygruppe;
- A: eine funktionelle Gruppe, die von einem bindenden Protein wie einem Antikörper oder Vitamin-D-bindendes Protein DBP mit hoher Affinität gebunden wird;
- R: die Seitengruppe eines Vitamin-D-Metabolits, bevorzugt die Seitengruppe von Vitamin-D₂ oder D₃, besonders bevorzugt die 25-hydroxylierte Seitengruppe von Vitamin-D₂ oder D₃.

Eine hohe Affinität liegt vor, wenn die Dissoziationskonstante (K) zwischen dem bindenden Protein, z.B. dem Antikörper oder DBP, und dem Antigen bzw. der funktionelle Gruppe A größer 10⁸ ist. Eine Dissoziationskonstante größer 10¹⁶ ist für viele Anwendungen vorteilhaft. In einer bevorzugten Ausführungsform ist A ausgewählt aus Biotin, Digoxigenin, Tyrosin, substituiertem Tyrosin, substituierten Aminosäuren, charakteristischen Aminosäure- und Peptidsequenzen, FITC, Proteinen und Proteingruppen wie Protein A und Protein G oder einem weiteren Vitamin-D-Derivat, ganz besonders bevorzugt 25-Hydroxyvitamin-D.

Die Abstandsgruppe X ist bevorzugt ausgewählt aus substituierten und unsubstituierten C-Körpern mit 0,8 bis 4,2 nm, bevorzugt etwa 0,12 nm Länge. Besonders bevorzugt ist eine Aminocarbonsäure, insbesondere eine Aminoundecansäure, peptidische und Ketogruppe oder ein substituierter oder unsubstituierter Aminopolyetherrest mit einer Länge von 0,8 bis 4,2 nm, bevorzugt etwa 0,9 bis 1,5 nm. Dieser Abstand zwischen der Gruppe A und der Bindungs- bzw. Erkennungsstelle für den Vitamin-D-Rest ist erforderlich, dass die bindenden Proteine an die jeweiligen Bindungsstellen binden können und dabei sich nicht gegenseitig stören. Zu beachten ist, dass beispielsweise für das Vitamin-D-bindende Protein DBP (Gc-Globlin) die 19-Methylengruppe, gegebenenfalls die 1-Hydroxygruppe des A-Rings und die Vitamin-D-Seitenkette zur Erkennungsstelle gehören und in einer Bindungstasche aufgenommen werden. Ähnliches gilt auch für spezifische Antikörper gegen die verschiedenen Vitamin-D-Derivate. Ist die Abstandsgruppe X zu kurz, kann neben dem Vitamin-D-bindenden Protein kein weiteres Bindungsprotein an der gewählten funktionellen Gruppe A binden. Für das bevorzugte Beispiel heißt das, wenn sich die funktionelle Biotin-Gruppe innerhalb der Bindungstasche des Vitamin-D-bindenden Proteins befindet, ist sie für das zweite Bindungsprotein, zum Beispiel das Streptavidin, nicht mehr zugänglich. Ist die Abstandsgruppe X hingegen zu lang, können Molekülfaltungen auftreten, welche gleichfalls die gleichzeitig Bindung zweier Bindungspartner behindern.

Daneben besitzt die erfindungsgemäße Abstandsgruppe überraschenderweise einen sterischen Effekt, als sie offenbar eine 180°-Drehung des A-Rings aktiv verhindern hilft. Es wird vermutet, ohne an diese Theorie gebunden zu sein, dass das 3β-Sauerstoffatom der Ethergruppe am A-Rings entsprechend einer natürlichen Hydroxygruppe hydratisiert ist und so einen Angriff auf die 5,6-Doppelbindung verhindert, abgesehen von anderen elektronischen und sterischen Effekten. Ein anderer wichtiger Aspekt ist, dass die Ethergruppe von den im Serum oder Plasma stets vorhandenen Esterasen nicht gespalten werden kann.

Ganz besonders bevorzugt ist 25-Hydroxyvitamin-D₃-3β-3'[6-N-(biotinyl)hexamido]amidopropylether der Formel II 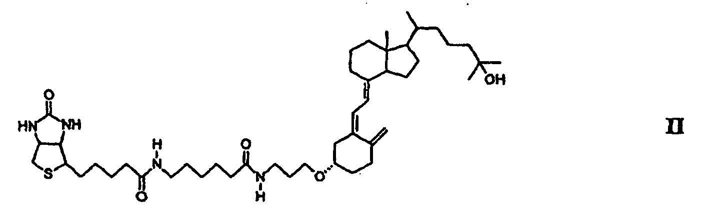 und die 1α-Hydroxy- und Vitamin-D₂-Analogen.

Weiterhin beschrieben sind Derivate, die als zweite funktionelle Gruppe einen Vitamin-D-Rest enthalten. Der Vorteil dieser Derivate ist, dass sie keine systemfremden Gruppen und Verbindungen enthalten und so eine erhöhte Empfindlichkeit und Zuverlässigkeit der kompetitiven Bindungsanalyse erlauben, auch weil sich eventuelle Bindungsbesonderheiten aus erster und zweiter Bindung des Vitamin-D-bindenden Proteins bei einer quantitativen Bestimmung kompensieren. Die Verbindungen besitzen nachstehende Formel III: 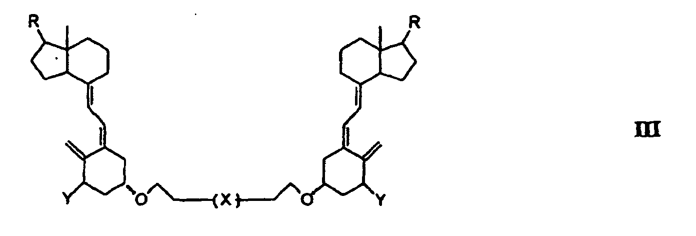 worin R, Y und X wie in oben in Formel I definiert sind. Besonders günstig sind dabei symmetrische Vitamin-D-Derivate.

Die 25-Hydroxy- und 1α,25-Dihydroxy-Vitamin-D-Derivate sind überraschend licht-, lager- und serumstabil und erlauben in allen kompetitiven immundiagnostischen Verfahren eine empfindliche, zuverlässige quantitative Bestimmung von Vitamin-D-Metaboliten wie 25-Hydroxy- und 1α,25-Dihydroxy-Vitamin-D, beispielsweise für die Routinediagnostik im Human- und Veterinärmedizinbereich sowie in der Forschung.

Erfindungsgemäß wird die Verbindung der Formel I erhalten durch ein Verfahren, umfassend die Schritte: a) Cyanoethylierung der 3-Hydroxygruppe von Vitamin-D oder 25-Hydroxyvitamin-D mit Acrylnitril in einem geeigneten Lösungsmittel wie Acetonitril in Gegenwart von Kaliumhydrid und tert.-Butanol; b) Reduktion der resultierenden Nitrilgruppe mit einem Gemisch aus Lithiumhydrid und Lithiumaluminiumhydrid zu einem Amin; und c) Anhängen einer Abstandsgruppe, gegebenenfalls zusammen mit einer funktionellen Gruppe A an das Amin, bspw. Biotinylieren der Verbindung mit einem aktivierten Biotinylierungsreagenz wie LC-BHNS oder, zum Erhalt eines Vitamin-D-Derivats gemäß Formel III, Kopplung von zwei Amino-Vitamin-D-Gruppen durch Kondensation mit einer Dicarbonsäure wie Sebacinsäure mittels Carbodiimid.

Das erfindungsgemäße Verfahren zur Herstellung funktioneller Vitamin-D-Derivate ergibt höhere Ausbeuten bei kürzeren Reaktionszeiten. Anders als in herkömmlichen Verfahren erfolgt nämlich in Schritt a) die Cyanoethylierung der 3-Hydroxygruppe in Gegenwart von Kaliumhydrid und tert.-Butanol. Dadurch wird erreicht, dass nur an der 3-Hydroxy-Gruppe von Vitamin-D eine Cyanoethylierung erfolgt und die andere Hydroxygruppen des Vitamin-D vor einer Umsetzung geschützt sind. Die Umsetzung erfolgt bei 0 bis 20°C, bevorzugt bei 5 bis 8°C in einem neutralen Lösungsmittel wie Acetonitril.

Bei der nachfolgenden Reduktion wird die Nitrilgruppe des Cyanoethylethers quantitativ in das Amin überführt, welches dann vergleichsweise einfach mit einer anderen funktionellen Gruppe verknüpft werden kann, zum Beispiel durch Umsetzung mit einem handelsüblichen Biotinylierungsreagenz.

Die Erfindung umfasst zudem die Verwendung der so hergestellten funktionellen Vitamin-D-Derivate in Verfahren zur Bestimmung von 25-Hydroxy- und 1α,25-Dihydroxy-Vitamin-D in Serum, Plasma, Urin oder einer anderen Probe. Hierbei wird das funktionelle Vitamin-D-Konjugat entweder als Zwischenglied eingesetzt, wobei das Vitamin-D-bindende Protein und nativer Vitamin-D-Metabolit um die Bindestelle kompetitieren, oder selbst als kompetitive Bindungskomponente zu nativem Vitamin-D. Das quantitative Bestimmungsverfahren ist vorzugsweise ein EIA (Enzym Immuno Assay), ELISA (Enzyme Linked Immuno Sorbent Assay), RIA(Radio Immun Assay), IRMA (Immuno Radio Metrischer Assay), LiA (Lumineszenz Immuno Assay), oder ILMA (Immuno Lumino Metrischer Assay), FIA (Fluoreszenz - Immuno Assay), oder IFMA (Immuno Fluoro Metrischer Assay) in manuell abzuarbeitenden Testsystemen oder auf Automaten angepasste Versionen in Flüssigphasen- wie Festphasentechnik.

Ein besonders bevorzugtes Verfahren zur Bestimmung von 25-Hydroxy-und 1α,25-Dihydroxy-Vitamin-D-Derivaten umfasst die Schritte: a) Beschichten eines Trägers mit Streptavidin, b) Zugabe ein oder mehrerer multifunktioneller Biotin-Vitamin-D-Derivate, c) Zugabe der Probe und einer definierten Menge Vitamin-D-bindendes Protein, d) Bestimmung des gebundenen Bindungsproteins mit markierten anti-Vitamin-D-Bindungsprotein-Antikörpern. Die Markierung der anti-Vitamin-D-Bindungsprotein-Antikörper kann direkt sein, bspw. eine radioaktive Markierung, oder auch indirekt, bspw. ein Enzym oder ein aktives Enzymfragment wie Peroxidase, das eine Farbreaktion zu katalysieren vermag.

Ein weiteres bevorzugtes Verfahren zur Bestimmung von 25-Hydroxy-und 1α,25-Dihydroxy-Vitamin-D-Derivaten umfasst die Schritte: a) Beschichten eines Trägers mit anti-Vitamin-D-Bindungsprotein-Antikörpern, b) Zugabe des Vitamin-D-Bindungsproteins, c) Zugabe der Probe sowie eine definierte Menge Biotin-Vitamin-D-Derivat, d) Bestimmung der Menge gebundenes Derivat mit markiertem Streptavidin. Das Streptavidin ist bevorzugt indirekt mit Peroxidase markiert; der Träger ist vorzugsweise eine Reaktionsgefäßwand, beispielsweise von einer Mikrotiterplatte, oder Teilchen aus polymerem oder magnetischem Material oder beidem, beispielsweise Kunststoff- oder Cellulose-Mikropartikel.

Diese Verfahren ermöglichen eine nicht-radioaktive quantitative Bestimmungen von 25-Hydroxy- und 1,25-Dihydroxy-Vitamin-D, ohne dass aufwendige Sicherheitsvorkehrungen erforderlich sind. Die hier vorgeschlagenen kompetitiven Verfahren sind somit für Routineuntersuchungen im Rahmen einer Osteoporose-Prophylaxe, bei einer vermuteten D-Hypovitaminose oder D-Hypervitaminose, zur Diagnostik allgemeiner Art und in der Forschung.

Ein weiterer Aspekt der Erfindung ist ein Kit zur Bestimmung von Vitamin-D-Metaboliten wie 25-Hydroxy- und 1,25-Dihydroxy-Vitamin-D, der unter anderem das erfindungsgemäße funktionelle Vitamin-D-Derivat enthält. Der Kit umfasst wahlfrei Vitamin-D-Bindungsprotein (Gc-Globulin), anti-Vitamin-D-Bindungsprotein-Antikörper, Streptavidin und vorbehandelte oder unvorbehandelte Mikrotiterplatten und/oder magnetische oder andere Mikropartikel und andere Reagenzien.

Weitere Vorteile, Merkmale und Ausführungsformen der Erfindung ergeben sich aus den nachstehenden Beispielen und den beiliegenden Zeichnungen. Es zeigt:
- **Fig.1**: den schematischen Syntheseweg für das bifunktionelle Vitamin-D-Derivat 25-Hydroxy Vitamin-D₃-3β-3'[6-N-(biotinyl)hexamido]amidopropylether) gemäß der Erfindung;
- **Figs. 2, 3** und **4**: Schemadarstellungen verschiedener ELISAs zur Bestimmung von 25-OH-Vitamin-D mit Hilfe des erfindungsgemäß hergestellten 25-OH-Vitamin-D-Konjugat;
- **Fig. 5A**: die Eichkurve eines kompetitiven ELISAs für 25-OH-Vitamin-D nach **Fig. 2**;
- **Fig. 5B**: Eichkurven von ELISAs gemäß **Fig. 5A** mit 3, 60 und 100 Tage altem 25-OH-Vitamin-D-Biotin-Tracer;
- **Fig. 5C**: die Eichkurve eines kompetitiven ELISAs für 1,25-Dihydroxy-Vitamin-D analog **Fig. 2**;
- **Figs. 6** und **7**: Schemadarstellungen kompetitiver RIA für 25-OH-Vitamin-D mit Hilfe des 25-OH-Vitamin-D-Konjugats;
- **Figs. 8, 9** und **10**: Schemadarstellungen kompetitiver, radioaktiver IRMAs für 25-OH-Vitamin-D mit Hilfe des 25-OH-Vitamin-D-Konjugats;
- **Figs. 11** und **12**: Schemadarstellungen kompetitiver ELISAs unter Verwendung von Mikropartikeln;
- **Fig. 13**: Schemadarstellung eines kompetitiven Bindungsassays für 25-OH-Vitamin-D mit Hilfe eines 25-OH-Vitamin-D-Konjugats und einem direktmarkierten Vitamin-D-bindenden Protein.
- **Fig. 14**: ein Blockdiagramm des Vergleichs der 1,25-Dihydroxy-Vitamin-D-Gehalte in Seren von Dialyse- und Normalpatienten.

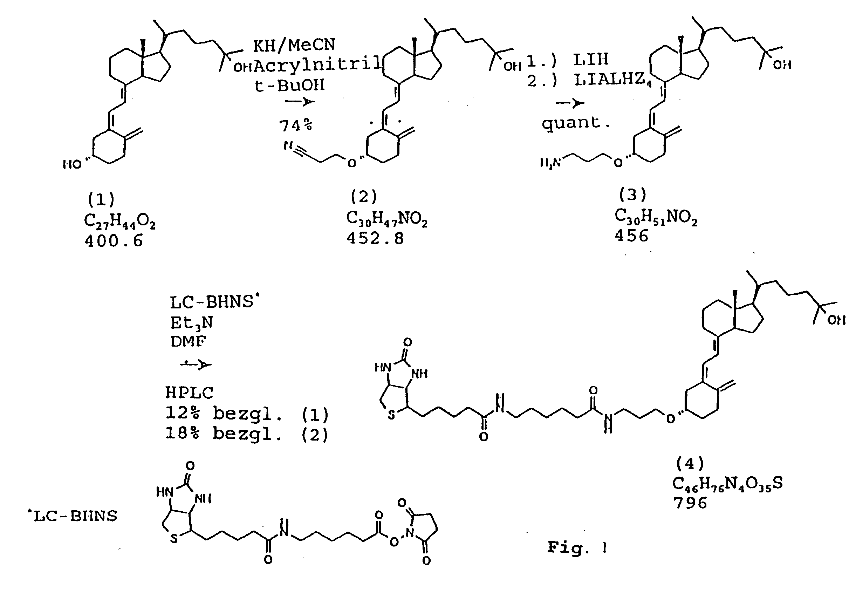

**Fig. 1** zeigt den erfindungsgemäßen Syntheseweg zur Herstellung des bifunktionellen 25-OH-Vitamin-D-Konjugats. Zunächst wird 25-OH-Vitamin-D in einem Gemisch aus Acetonitril, Kaliumhydrid und tert.-Butanol mit Acrylnitril cyanoethyliert. Durch die Gegenwart des als Base fungierenden Kaliumhydrids und durch die Anwesenheit von tert.-Butanol zur Abwendung unspezifischer Reaktionen an der 25-Hydroxygruppe wird erreicht, dass selektiv die 3-Hydroxy-Gruppe des Vitamin-D. cyanoethyliert wird. Die Ausbeute an 25-OH-Vitamin-D-3β-cyanoethylether beträgt in der Regel etwa 74% bei einer Reaktionsdauer von 40 Minuten.

Nach üblicher Aufarbeitung wird der 25-OH-Vitamin-D-3β-cyanoethylether mit Lithiumhydrid versetzt und die 25-Hydroxygruppe in das Lithiumalkoholat überführt. Es folgt eine Reduktion des Nitrils mit LiAlH₄ zu 25-OH-Vitamin-D-3β-3'-aminopropylether. Dieser Schritt ist quantitativ, ohne dass Nebenprodukte auftreten. Zuletzt erfolgt gegebenenfalls eine Biotinylierung mit einem aktivierten Biotinylierungsreagenz wie LC-BHNS (Biotinyl-N-ε-aminocaproyl-hydroxysuccinimidester). Die resultierende Abstandsgruppe X hat entsprechend der Aminocaproylkette eine Länge von etwa 0.8 bis 0.9 nm.

25-OH-Vitamin-D-3β-3'[6-N-(biotinyl)hexamido]amidopropylether ist temperaturstabil und kann in einer wässrigen, leicht sauren Matrix über mehrere Monate gelagert werden. Da die Verbindung von Serumenzymen nicht gespalten wird, eignet sie sich bestens für diagnostische Routinetests in Serum, Plasma und Urin.

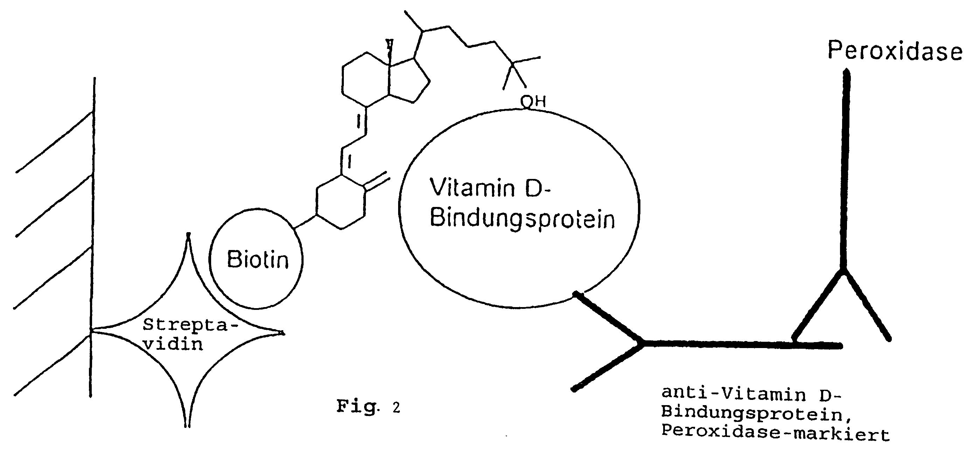

**Fig. 2** zeigt eine Schemadarstellung eines kompetitiven ELISA für 25-OH-Vitamin-D. Hierbei wird das 25-OH-Vitamin-D-Konjugat (25-OH-Vitamin-D-3β-3'[6-N-(biotinyl)hexamido]amidopropylether) über Streptavidin an eine feste Phase gebunden. Dann erfolgt in flüssiger Phase die kompetitive Bindung von Vitamin-D-bindenden Protein und 25-OH-Vitamin-D aus Standard oder Probe an das 25-OH-Vitamin-D-Konjugat. Der Nachweis erfolgt durch Peroxidase-markierte Antikörper gegen das Vitamin-D-bindende Protein. Der Fachmann weiß, dass auch andere Markerenzyme verwendet werden können, zum Beispiel alkalische Phosphatase oder Galaktosidase, etc.

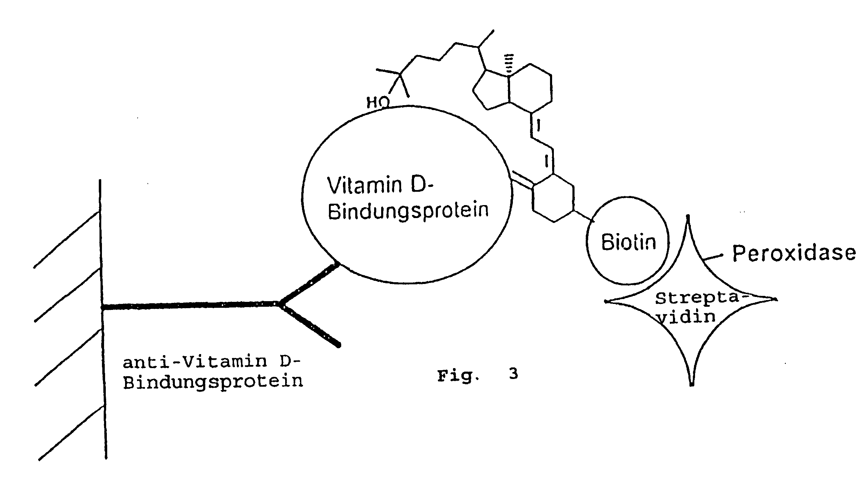

**Fig. 3** zeigt die Schemadarstellung eines kompetitiven, nicht-radioaktiven ELISA, wobei das Vitamin-D-bindende Protein zunächst über anti-Vitamin-D-Bindungsprotein-Antikörper an die feste Phase gebunden wird. Danach erfolgt in flüssiger Phase die kompetitive Bindung von 25-OH-Vitamin-D-Biotin und 25-OH-Vitamin-D aus Standard bzw. Probe. Zur Bestimmung wird dann Peroxidase-markiertes Streptavidin eingesetzt. Das gezeigte Prinzip lässt sich selbstverständlich auf andere Tracer-Gruppen statt Biotin und auf andere Markerenzyme, wie oben angegeben, übertragen.

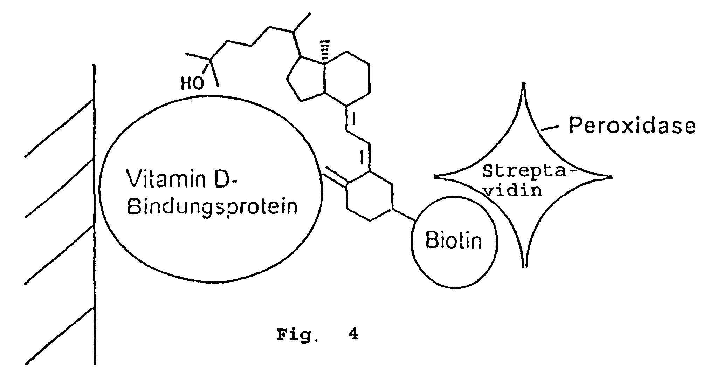

**Fig. 4** zeigt die Schemadarstellung eines kompetitiven ELISA, wobei das Vitamin-D-bindende Protein direkt an die feste Phase gebunden ist. Die kompetitive Bindung von 25-OH-Vitamin-D₃-Biotin und 25-OH-Vitamin-D₃ aus Standard bzw. Probe erfolgt in flüssiger Phase und Peroxidase-markiertes Streptavidin wird zur quantitativen Bestimmung eingesetzt.

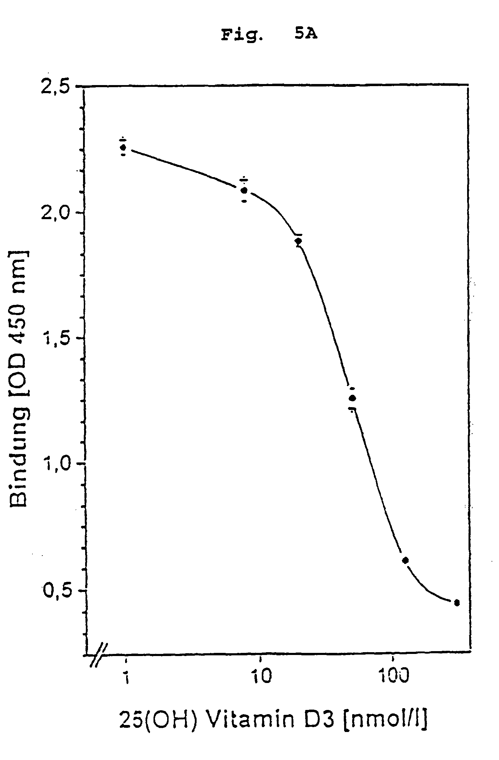

**Fig. 5A** -C zeigen die typische Eichkurven von kompetitiven ELISAs mit 25-OH-bzw 1,25-Dihydroxy-Vitamin-D₃-Biotin gemäß dem in **Fig. 2** gezeigten Prinzip. Die Menge an gebundenem Vitamin-D-Bindungsprotein wurde bestimmt durch eine standardisierte Farbreaktion mit Peroxidase-gekoppelten anti-Vitamin-D-Bindungsprotein-Antikörpern und Tetramethylbenzidin (TMB) als Substrat. Alternative Substrate wären beispielsweise OPD (1,2-Phenyldiamin x 2 HCl), ABTS und andere. Für die Eichkurve wurden Vitamin-D-Proben mit Konzentrationen von 0, 8, 20, 50, 125 und 312 nMol/l eingesetzt. Die Ordinate zeigt die optische Dichte als Mittelwert von zwei Messungen bei 450 nm; die Abszisse die Konzentration an 25-OH- bzw. 1,25-Dihydroxy-Vitamin-D in nMol/l.

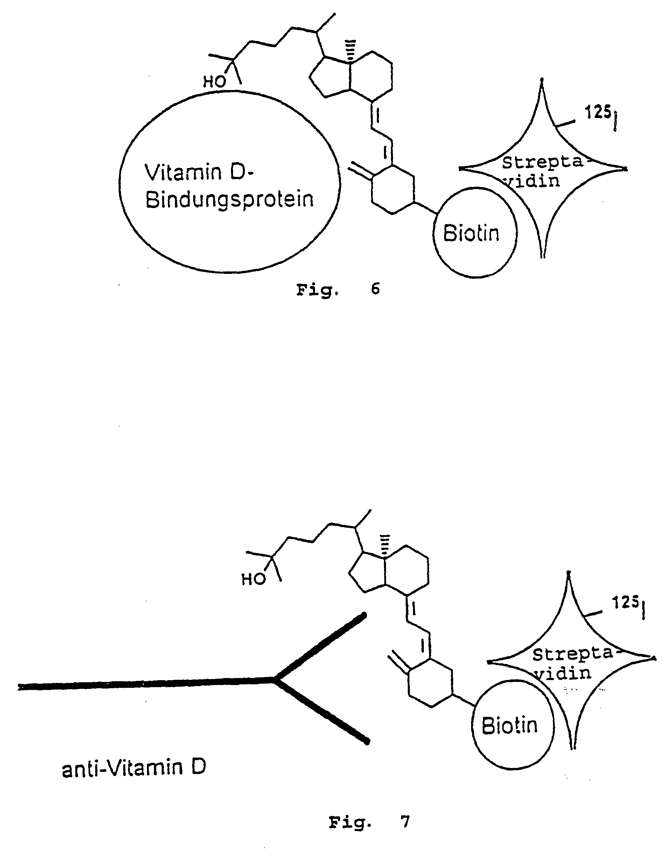

**Fig. 6** zeigt die Schemadarstellung eines kompetitiven Protein-Bindungsversuch (CPBA), wobei 25-OH-Vitamin-D₃-Biotin und 25-OH-Vitamin-D aus Standard bzw. Probe in flüssiger Phase um die Bindungsstelle des Vitamin-D-Bindungsproteins kompetitieren. Es wird ¹²⁵I-markiertes Streptavidin für die quantitative Bestimmung verwendet.

**Fig. 7** zeigt die Schemadarstellung eines kompetitiven Radioimmunoassays (RIA), wobei 25-OH-Vitamin-D-Biotin und 25-OH-Vitamin-D aus Standard bzw. Probe in flüssiger Phase um die Bindungsstelle eines anti-Vitamin-D-Antikörpers kompetitieren. Für die quantitative Bestimmung wird ¹²⁵I-markiertes Streptavidin eingesetzt. Erfolgt die Bestimmung durch ein Streptavidin, das nicht radioaktiv, sondern mit einem Fluorophor oder Luminophor markiert ist, liegen sogenannte LIA- oder FIA-Assays vor.

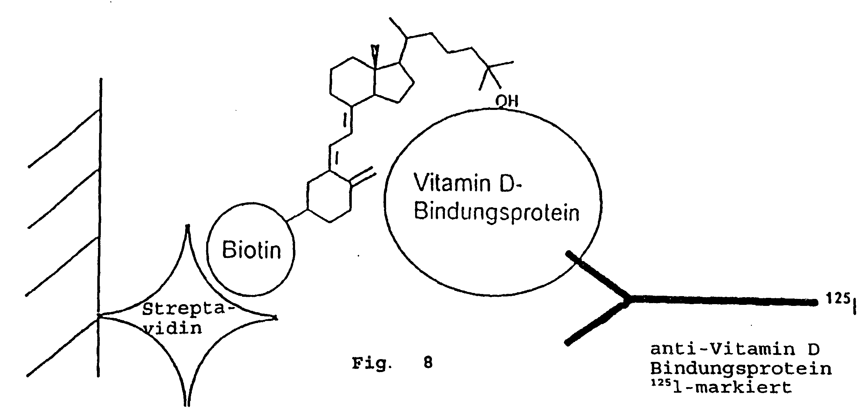

**Fig. 8** zeigt die Schemadarstellung eines 25-OH-Vitamin-D-IRMA. Es wird zunächst 25-OH-Vitamin-D-Biotin über Streptavidin an die feste Phase gebunden. Die kompetitive Bindung von Vitamin-D-Bindungsprotein an das Konjugat und 25-OH-Vitamin-D₃ aus Standard oder Probe erfolgt dann in flüssiger Phase. Die Menge des Konjugat-gebundenen Bindungsproteins wird mit ¹²⁵I-markierten Antikörpern bestimmt.

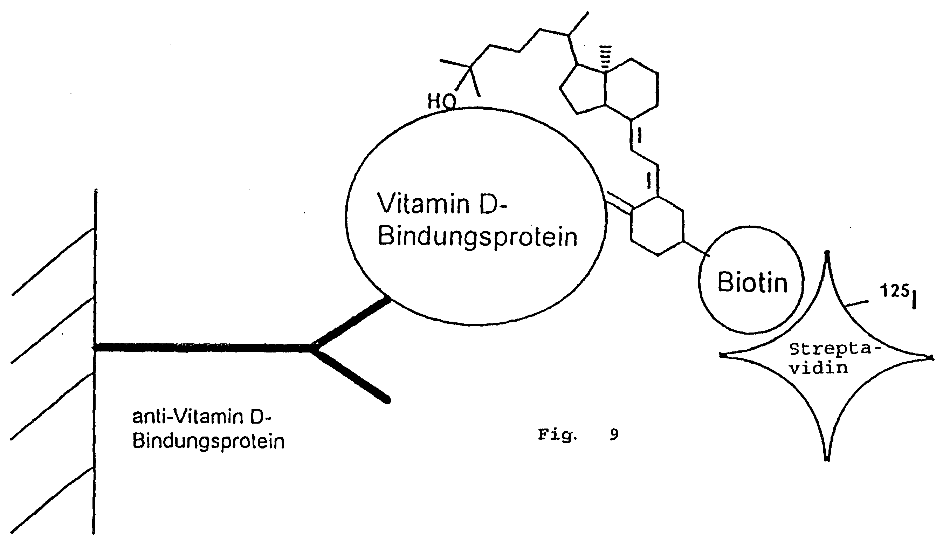

**Fig. 9** ist die Schemadarstellung einer IRMA-Sandwich-Technik (Immunoradiometrischer Assay). Hierzu werden anti-Vitamin-D₃-Antikörper an die feste Phase gekoppelt. An diese binden dann Vitamin-D-Bindungsproteine. Die Kompetition erfolgt im nächsten Schritt zwischen dem 25-OH-Vitamin-D-Konjugat und 25-OH-Vitamin-D aus Standard oder Probe. Die Bestimmung der Menge gebundenes Konjugat erfolgt durch ¹²⁵I-markiertes Streptavidin.

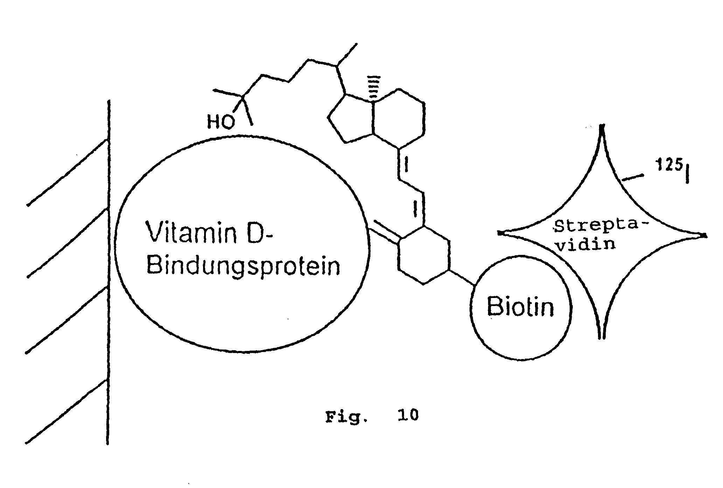

**Fig. 10** zeigt die Schemadarstellung eines weiteren IRMA-Sandwich-Technik. Es wird zunächst Vitamin-D₃-Bindungsprotein an die feste Phase gekoppelt. Dann erfolgt daran die kompetitive Bindung zwischen dem 25-OH-Vitamin-D₃-Konjugat und 25-OH-Vitamin-D₃ aus Standard oder Probe. Die Menge gebundenes Konjugat wird durch ¹²⁵I-markiertes Streptavidin bestimmt.

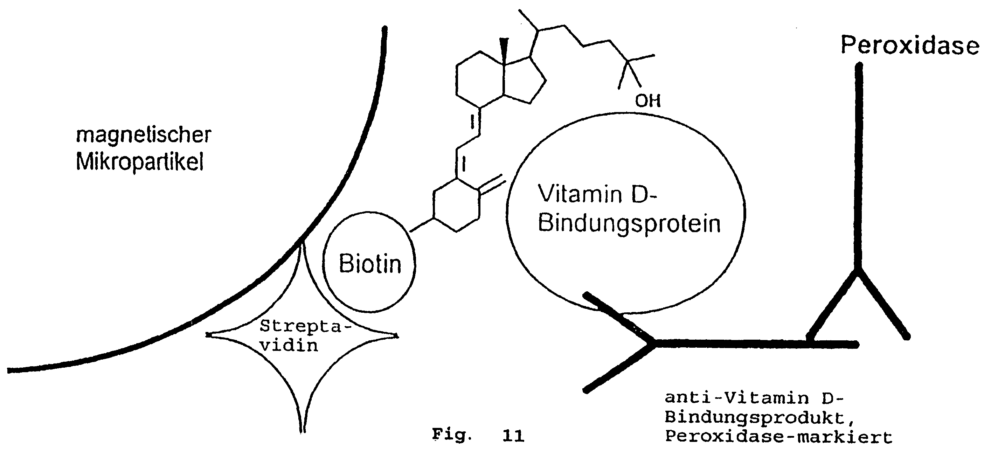

**Fig. 11** zeigt die Schemadarstellung eines kompetitiven ELISA unter Verwendung von Mikropartikeln. Hierbei wird 25-OH-Vitamin-D-Biotin über Streptavidin an Mikropartikel gebunden. Dann wird 25-OH-Vitamin-D-Derivat daran gebunden. In flüssiger Phase wird Vitamin-D-Bindungsprotein und die jeweilige Probe zugegeben. Bindungsproteine und 25-OH-Vitamin-D₃ aus Standard oder Probe kompetitieren um die Bindungsstelle des Konjugats. Die gebundenen Bestandteile werden dadurch abgetrennt, dass sie über die Mikropartikel von einem Magneten zurückgehalten werden, während der Überstand mit den ungebundenen Substanzen entfernt wird. Die Menge gekoppeltes Bindungsprotein wird bestimmt in einem zweistufigen Verfahren mit einem primären Antikörper gegen Vitamin-D-Bindungsprotein und einem sekundären Peroxidase-markierten Antikörper.

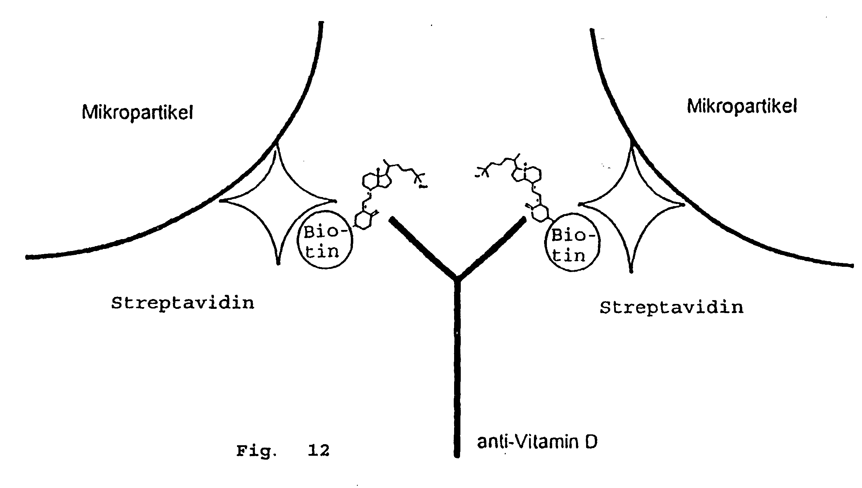

**Fig. 12** zeigt eine Schemadarstellung eines kompetitiven ELISA unter Verwendung von Mikropartikeln. Es wird 25-OH-Vitamin-D-Biotin über Streptavidin an Mikropartikel gebunden. Dann wird die flüssige Probe mit 25-OH-Vitamin-D₃ (aus Standard oder Probe) dazugegeben sowie eine nicht-sättigende Menge Antikörper. Das Konjugat und das native Vitamin-D₃ kompetitieren um die Bindung des Antikörpers. Die Menge gebundener Antikörper erfolgt durch Agglutination der Mikropartikel. Diese kann beispielsweise direkt durch Nephelometrie bzw. Turbimetrie bestimmt werden.

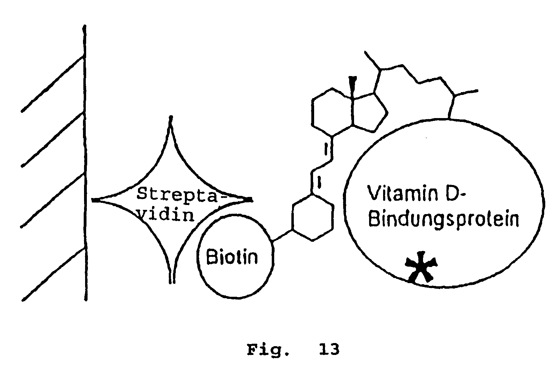

**Fig. 13** zeigt das Schema eines kompetitiven Bindungsassays, wobei das Vitamin-D-bindende Protein direkt markiert ist, beispielsweise radioaktiv mit ¹²⁵Iod, oder für eine Elektrochemolumineszenz mit Ruthenium(II)tris-(bipyridin)-NHS-ester. Die Markierung können auch Enzyme sein wie Peroxidase, alkalische Phosphatase, β-Galactosidase u.a., oder auch FITC.

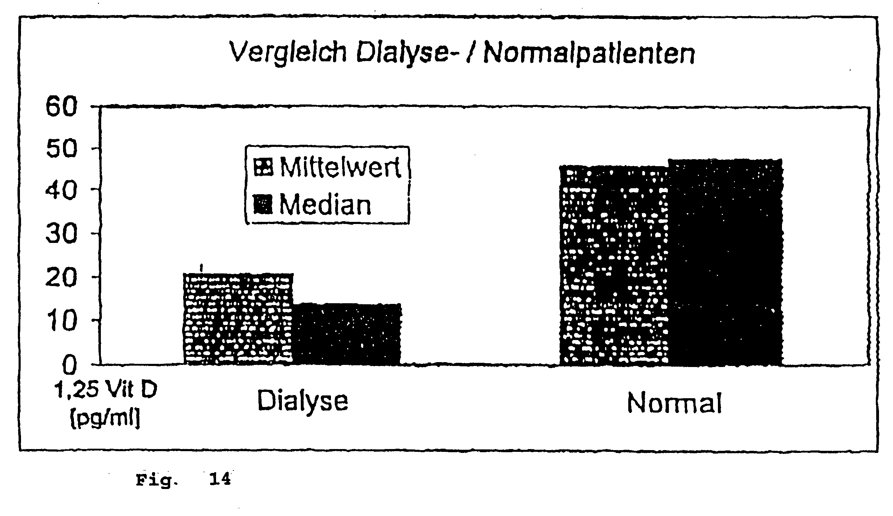

**Fig. 14** veranschaulicht in einem Blockdiagramm die unterschiedlichen 1,25-Dihydroxy-Vitamin-D-Gehalte im Serum von Dialyse- und Normalpatienten.

Die bekannten Bestimmungsverfahren für Proteine wie der kompetitive ELISA beruhen auf dem Prinzip, dass die nachzuweisende Verbindung mit einem Bindungsprotein oder Konjugat um eine Bindungsstelle kompetitiert. Es wird dann die Menge gebundenes Bindungsprotein oder Konjugat bestimmt und anhand einer Eichkurve die Konzentration der nachzuweisenden Verbindung ermittelt.

Die in den Figuren gezeigten Testprinzipien lassen sich einfach auf andere Vitamin-D-Derivate übertragen. Besonders erwähnt sei 1α,25-Dihydroxy-Vitamin-D₂/D₃. In diesem Fall muss ein Bindungsprotein bzw. ein Rezeptor oder Antikörper gewählt werden, der das 1α,25-Dihydroxy-Vitamin-D-Analoge spezifisch erkennt. Das zugehörige bifunktionelle 1α,25-Dihydroxyderivat kann gewonnen werden enzymatisch durch Umsetzung von 25-OH-Vitamin-D-3β-cyanoethylether mit 25-OH-Vitamin-D-1α-Hydroxylase, Reduktion zum Amin und schließlich Addition der zweiten funktionellen Gruppe. Ferner werden hier Derivate von Vitamin-D₂ und Vitamin-D₃ vorgeschlagen. Deren Synthese kann auf dem in Beispiel 1 vorgestellten Weg erfolgen

### BEISPIELE

### Beispiel 1: Synthese von 25-OH-Vit.-D₃-3β-3'[6-N-(biotinyl)hexamido]amidopropylether (4)

Alle Umsetzungen erfolgten im Dunkeln unter trockener Stickstoffatmosphäre. Zwischenprodukte wurden bei -20°C gelagert. Es wurden HPLC-reine Lösungsmittel eingesetzt. Das 25-OH-Vitamin-D₃ war von BIOMOL Feinchemikalien GmbH, Hamburg, der LC-BHNS (Long-Chain-Biotinyl-N-ε-aminocaproyl-hydroxysuccinimidester) von Sigma Chemie, alle weiteren Chemikalien von Fluka, Darmstadt. Die Massenspektroskopie(FAB) erfolgte mit einem Finigan-MAT-90, die NMR-Messungen mit einem Bruker-ARX-400 (400 MHz) oder einem Bruker-ARC-250F (250 MHz).

### (i) 25-OH-Vitamin-D₃-3β-cyanoethylether (2)

5 mg 25-OH-Vitamin-D₃ (12,5 µMol), gelöst in Methylenchlorid (CH₂Cl₂), wurde in einen mit Stickstoff gefüllten Kolben überführt und das Lösungsmittel abgezogen. Der feste Rückstand wurde in 1 ml Acetonitril aufgenommen und versetzt mit 10 Tropfen einer Mischung von tert.-Butanol und Acetonitril (9:1 v/v) sowie 130 µMol Acrylnitril (10 eq.) in 100 µl Acetonitril [Stammlösung: 86 µl Acrylnitril (1.3 mMol) verdünnt mit Acetonitril auf 1 ml]. Die klare Lösung wurde 15 Minuten bei 6°C gerührt. Es wurde 625 µMol Kaliumhydrid (0,5 eq.) in 25 µl tert.-Butanol/Acetonitril (9:1 v/v) [Stammlösung: 10 mg KH (250 µMol) in 1 ml tert.-Butanol/Acetonitril (9:1 v/v)] zugegeben. Die dabei entstehende Ausflockung löste sich sofort wieder. Die Mischung wurde bei 6°C gerührt. Die wiederholte Dünnschichtchromatographie (DC) einzelner Proben mit 20% Petrolether in Methyl-tert.-butylether (MTBE) auf Kieselgel zeigte, dass nach 10 Minuten bereits 90% der Ausgangsverbindung umgesetzt waren. Nach 15 Minuten wurden wenige Tropfen Reaktionsgemisch mit etwa 5 Tropfen Wasser und 0,5 ml MTBE aufgearbeitet. Die DC der organischen Phase zeigte kein Edukt mehr. Nach 40 Minuten wurde das gesamte Reaktionsgemisch mit Wasser/MTBE aufgearbeitet. Es wurde 4 mg öliges Produkt erhalten.

| | | |
|---|---|---|
| IR (NaCl/CH₂Cl₂): | 3422 | OH |
| | 2941,2872 | CH |
| | 2252 | Nitril |
| | 1105 | Ether |

Die HPLC-Analyse (3% MeOH/CH₂Cl₂) ergab 93% Produkt und 7% Edukt. 4 mg Produkt enthielten somit 3.7 mg (8,2 µMol) Zielverbindung, was einer Ausbeute von 74% entspricht.

### (ii) 25-OH-Vitamin-D₃-3β-3'aminopropylether (3)

3.75 mg (8.3 µMol) Nitril aus (i) wurde in 2 ml Ether gelöst, mit 125 µMol Lithiumhydrid gelöst in 1 ml Ether (Stammlösung: 7 mg frisches feinpulvriges LiH in 7 ml Ether) versetzt und eine Stunde bei Raumtemperatur unter Stickstoff gerührt. Es wurde 169 µMol LiAlH₄ als Aufschlämmung in 1 ml Ether (Stamm: 18 mg frisches feinpulvriges LiAlH₄ in 3 ml Ether) zugegeben. Nach einer weiteren Stunde wurde das Gemisch mit 1 ml konzentriertem KOH, 5 ml H₂O und 4 x 20 ml MTBE aufgearbeitet. Die Dünnschichtchromatographie einer Probe mit 1:1 MTBE/Petrolether auf Kieselgel zeigte nur noch den Ausgangspunkt. Das Diol lag bei Rᵢ 0.27; das Nitril bei Rᵢ 0.4. Die gewonnene Substanz wurde ohne weitere Analyse und Aufreinigung weiterverarbeitet.

### (iii) 25-Hydroxyvitamin-D₃-3β-3'[6-N-(biotinyl)hexamido]amidopropylether (4)

Es wurde 3 mg (6.6 µMol) 25-OH-Vitamin-D₃-3β-aminopropylether (3) aus (ii) in 1 ml Dimethylformamid (DMF) gelöst. Dann wurde unter Stickstoff 3 mg (6.6 µMol) LC-BNHS und 1 µl (17.5 µMol) Triethylamin zugegeben. Es wurde 18 h bei Raumtemperatur gerührt, das DMF abgezogen und der Rückstand mit 20% Methanol (MeOH) in CH₂Cl₂ vorgereinigt. 12 mg (> 100%) der so gewonnenen Substanz wurden mittels HPLC gereinigt (Bedingungen: Knauer Kromasil-100, 5 µM, 250 x 4 mm, 10% MeOH in CH₂Cl₂, 1,5 ml/min, OD 265 nm, 7 Minuten). Die Ausbeute betrug 1,2 mg (1,5 µMol). Dies entspricht 12%, bezogen auf das 25-OH-Vitamin-D₃ und 18% bezogen auf die Nitrilverbindung.

**Tabelle I**

| **Biotin-25-OH-Vitamin-D**_{**3**} | | | | |
|---|---|---|---|---|
| | H | Mult | cc [Hz] | Zuordnung |
| 6,42 | 1 | Dd | 5,7 | NH (Biotin) |
| 6,2 | 1 | D | 11 | 6 |
| 6,0 | 1 | D | 11 | 7 |
| 5,85 | 1 | Dd | 5,7 | NH (Biotin) |
| 5,55 | 2 | M | | 3-O-CH₂(28) |
| 5.38 | 1 | S | | NH oder OH |
| 5,05 | 1 | D | 2 | 19 |
| 4,83 | 1 | D | 2 | 19 |
| 4,77 | 1 | S | | NH oder OH |
| 4.51 | 1 | M | | HC-NH I Biotin |
| 4,33 | 1 | M | | HC-NH II Biotin |
| 3,53 | 1 | M | | 3 |
| 2,53 | 1 | D | 10 | 4 |
| 1,21 | 6 | S | | 26,27-CH₃ |
| 0,93 | 3 | D | 6 | 21-CH₃ |
| 0,54 | 3 | S | | 18-CH₃ |
| **MS** (Finigan MAT 90); (FAB): 797 (MH⁺) vom 5.9.97 und 28.11.97; ¹**H-NMR** (Bruker ARX 400) in CDCl₃/TMS bei 400 MHz. Die Analysedaten sind in Tabelle I gezeigt. | | | | |

### Beispiel 2: Stabilität von 25-OH-Vit.-D₃-3β-3'[6-N-(biotinyl)hexamido]amidopropylether

Es wurde jeweils 20 mg gereinigte 25-OH-D₃-Biotin-Verbindung (25-OH-Vitamin-D₃-3β-3'[6-N-(biotinyl)-hexamido]amidopropylether) aus Beispiel 1 in ein NMR-Röhrchen gegeben und mit 1 ml Lösungsmittel versetzt. Das Lösungsmittel war eine Mischung aus Deuteriochloroform:Deuteroacetonitril:D₂O im Verhältnis 3:2:1 mit einem pH-Wert zwischen 4 und 5. Die Proben wurden 200 Tage unter nachstehenden Bedingungen gelagert und in regelmäßigen Abständen die NMR-Spektren untersucht.
- Probe 1:: unter Lichtausschluss bei - 20°C;
- Probe 2:: unter Lichtausschluss bei +4-6°C;
- Probe 3:: unter Lichtausschluss bei Umgebungstemperatur;
- Probe 4:: unter starker Lichteinwirkung (auf der Fensterbank) bei Umgebungstemperatur.

Die Proben 1 und 2 zeigten über die ganzen Zeit keine wesentlichen Veränderungen im NMR-Spektrum. Eine HPLC-Analyse bestätigte, dass die Proben 1 und 2 auch nach 200 Tagen protisches Lösungsmittel intakt waren. Probe 3 zeigte eine minimale Veränderung im NMR-Spektrum nach 100 Tagen. Die HPLC-Analyse ergab, dass mehr als 78% der Verbindung noch intakt war. Probe 4 war nach 2 Monaten abgebaut. Die Stabilitätsuntersuchung zeigt, dass die Verbindung unter Ausschluss von Licht sehr beständig ist, selbst in protischen Lösungsmitteln und ohne Kühlung.

### Beispiel 3: 25-Hydroxyvitamin-D-ELISA mit 25-Hydroxyvitamin-D₃-3β-3'[6-N-(biotinyl)hexamido]amidopropylether

Die Bestimmung erfolgte nach dem in **Fig. 2** gezeigten Prinzip. Hierzu musste zunächst 25-OH-Vitamin-D-3β-3'[6-N-(biotinyl)hexamido]amidopropylether über Streptavidin an eine feste Phase gebunden werden.

### (i) Beschichtung einer Mikrotiterplatte mit Streptavidin

In die Vertiefungen einer Mikrotiterplatte wurde jeweils 100 ng Streptavidin gegeben, gelöst in 200 µl 60 mM NaHCO₃, pH 9,6, und die Platte über Nacht bei 4°C inkubiert. Die Streptavidin-Lösung in den Vertiefungen wurde entfernt und jede Vertiefung fünfmal mit 200 µl Waschpuffer (PBS, pH 7.4 mit 0.05% Tween-20) gewaschen. Dann wurde in jede Vertiefung 250 µl Assaypuffer gegeben. Für den Assaypuffer wurde 5 g Casein in 100 ml 0, 1 N NaOH gelöst und mit PBS, pH 7,4 auf 1 L Volumen aufgefüllt. Die Lösung wurde eine Stunde gekocht, das Volumen mit destilliertem Wasser auf einen Liter ergänzt, der pH-Wert auf 7,4 eingestellt und 0,1 g Thimerosal zur Vermeidung von Mikrobenwachstum zugefügt. Die Vertiefungen in der Mikrotiterplatte wurden eine Stunde bei Raumtemperatur mit Assaypuffer inkubiert, dann der Assaypuffer entfernt und jede Vertiefung fünfmal mit je 200 µl Waschpuffer gewaschen.

### (ii) Bindung von 25-Hydroxyvitamin-D₃-3β-3'[6-N-(biotinyl)hexamido]amidopropylether

Es wurde in eine jede Vertiefung 100 µl Biotin-Vitamin-D-Lösung gegeben (10 ng 25-OH-Vitamin-D-3β-3'[6-N-(biotinyl)hexamido]amidopropylether in 100 µl Waschpuffer) und eine Stunde bei Raumtemperatur im Dunkeln und unter Schütteln inkubiert. Dann wurde die Biotin-Vitamin-D-Lösung aus den Vertiefungen entfernt und jede Vertiefung fünfmal mit je 200 µl Waschpuffer gewaschen. Es folgte in flüssiger Phase die kompetitive Bindung von Vitamin-D-bindenden Protein in Gegenwart von 25-OH-Vitamin-D aus Standard oder Probe.

### (iii) Probenvorbereitung

Es wurde 50 µl Serum in einem 1,5 ml Eppendorf-Reaktionsgefäß mit 200 µl Ethanol_{abs} (vorgekühlt auf -20°C) durch Vortexen gemischt und 20 Minuten bei -20°C ausgefällt. Die Proben wurden in einer Eppendorf-Tischzentrifuge bei maximaler Umdrehung zentrifugiert und die Überstände abgenommen und im ELISA eingesetzt.

Man kann in der Regel davon ausgehen, dass Plasma oder Serumproben bei 4°C etwa 2 Wochen stabil sind. Bei längerer Lagerung müssen sie bis zur Bestimmung tiefgefroren werden. Urinproben müssen vor einer Lagerung mit 1 N NaOH auf einen pH-Wert zwischen 6 und 8 eingestellt werden. Sie können dann bei 4°C etwa 14 Tage aufbewahrt werden; bei längerer Lagerung müssen auch sie bis zur Bestimmung tiefgefroren werden.

### (iv) Kompetitive Bindung

Es wurde jeweils 100 µl Vitamin-D-bindendes Protein, isoliert aus Ziegenserum (1:15000 in Assaypuffer mit 3% (w/v) PEG 6000), zusammen mit 10 µl Standard, Kontrolle oder Probe (10 µl Überstand aus der Probenvorbereitung) in die Vertiefungen gegeben. Die Mikrotiterplatte wurde 24 Stunden bei 4°C im Dunkeln und unter Rütteln inkubiert. Dann wurden die Lösungen aus den Vertiefungen entfernt und die Vertiefungen fünfmal mit je 200 µl Waschpuffer gewaschen.

### (v) Bestimmung der kompetitiven Bindung

Es wurde jeweils 100 µl Kaninchen-anti-Vitamin-D-Bindungsprotein (1:10000 verdünnt in Assaypuffer mit 3% (w/v) PEG 6000) in die Vertiefungen gegeben und eine Stunde im Dunkeln und unter Rütteln bei Raumtemperatur inkubiert. Die Lösungen wurden aus den Vertiefungen entfernt und jede Vertiefung fünfmal mit je 200 µl Waschpuffer gewaschen. Die quantitative Bestimmung erfolgte mit 100 µl anti-Kaninchen-IgG-Peroxidase (1:20 000 verdünnt in Waschpuffer). Es wurde eine Stunde bei Raumtemperatur inkubiert. Danach wurden Antikörper-Lösungen abgenommen und eine jede Vertiefung fünfmal mit je 200 µl Waschpuffer gewaschen. Für die Farbreaktion wurden 100 µl Tetramethylbenzidin(TMB)-Substrat-Lösung (gebrauchsfertig von NOVUM Diagnostika GmbH, Dietzenbach, DE) in die Vertiefungen gegeben. Nach 30 Minuten wurde die Farbentwicklung gestoppt durch Zugabe von 50 µl 2 M H₂SO₄ pro Vertiefung. Die Messung der optischen Dichte erfolgte bei 450 nm. Die nachstehenden Tabellen II und III zeigen das Pipettierschema für die Mikrotiterplatte sowie die Werte für die optische Dichten.

Als Standard wurden Lösungen von 25-OH-Vitamin-D₃ in Assaypuffer mit folgenden Konzentrationen eingesetzt: 0, 8, 20, 50, 125 und 312 nMol/L (siehe Eichkurve in **Fig. 5A**). Als Kontrolle bzw. Proben dienten vier Seren von Patienten mit einer D-Hypovitaminose (Proben-Nrn. 24, 203, 963, 965) und sowie vier willkürlich ausgewählte Normalseren (Proben-Nm. NP 18, NP 25, NP 34, NP 37 - Testreihen 3 und 4). Von den Vitamin-D-Mangelseren wurde zudem die 25-OH-Vitamin-D-Konzentration durch kompetitiven Bindungsassay mit Hilfe von ³H-25-OH-Vitamin-D bestimmt. Als weitere "Kontrolle" dienten vier Lösungen, bei denen die jeweilige Konzentration an 25-OH-Vitamin-D aus anderweitigen Bestimmungen bekannt war, entweder aus den Herstellerangaben oder durch einen kompetitiven Bindungsassay (CBPA) mit ³H-25-OH-Vitamin-D.

**Tabelle II**

| Probenanordnung | | | | | | |
|---|---|---|---|---|---|---|
| **Pipettier Schema** | Standard nMol / L | Doppelwert von Spalte 1 | Serumprobe Nr. | Doppelwert von Spalte 3 | Kontrollen | Doppelwert von Spalte 5 |
| Reihe / Spalte | **1** | **2** | **3** | **4** | **5** | **6** |
| **A** | NSB | NSB | 24 | 24 | K1 (CBPA) | K1 (CBPA) |
| **B** | 0 | 0 | 203 | 203 | K2 (CBPA) | K2 (CBPA) |
| **C** | 8 | 8 | 963 | 963 | K3 (HPLC) | K3 (HPLC) |
| **D** | 20 | 20 | 965 | 965 | K4 (HPLC) | K4 (HPLC) |
| **E** | 50 | 50 | NP 18 | NP 18 | | |
| **F** | 125 | 125 | NP 25 | NP 25 | | |
| **G** | 312 | 312 | NP 34 | NP 34 | | |
| **H** | | | NP 37 | NP 37 | | |
| NSB: Nichtspezifischer Bindungspuffer (Assaypuffer ohne Vitamin-D-Bindungsprotein) | | | | | | |

**Tabelle III**

| Messwerte nach 30 Minuten Farbentwicklung | | | | | | |
|---|---|---|---|---|---|---|
| **OD 450 nm** | Standard | Doppelwert zu Spalte 1 | Serumprobe Nr. | Doppelwert zu Spalte 3 | Kontrollen | Doppelwert zu Spalte 5 |
| Reihe / Spalte | **1** | **2** | **3** | **4** | **5** | **6** |
| **A** | --- | --- | 0.947 | 1.023 | 1.903 | 2.300 |
| **B** | 2.256 | 2.182 | 0.853 | 0,910 | 0.393 | 0.371 |
| **C** | 1.845 | 1.861 | 0.646 | 0.637 | 1.674 | 1.586 |
| **D** | 1.432 | 1.456 | 1.429 | 1.303 | 0.578 | 0.634 |
| **E** | 0.625 | 0.612 | 0.524 | 0.547 | | |
| **F** | 0.287 | 0.261 | 0.454 | 0.419 | | |
| **G** | 0.156 | 0.176 | 0.341 | 0.368 | | |
| **H** | --- | --- | 0.421 | 0.386 | | |
| Bₘₐₓ | 2.801 | 2.676 | | | | |

Aus den Mittelwerten der Spalten 1 und 2 und den bekannten Konzentration an 25-OH-Vitamin-D wurde die in **Fig. 5A** gezeigt Eichkurve erstellt. Die Ordinate zeigt die optische Dichte als Mittelwert der beiden Messungen bei 450 nm; die Abszisse die Konzentration an 25-OH-Vitamin-D in nMol/l. Die Ergebnisse sind in der Tabelle 5 zusammengefasst.

### Beispiel 4: Vergleichende Bindungsanalyse mit ³H-25-OH-Vitamin-D als kompetitiver Partner

Soweit nicht anders angegeben ist, waren alle Reagenzien Puffer und Materialien gleich wie im vorgenannten Beispiel 3. Als kompetitiver Bindungspartner (Tracer) diente Tritium-markiertes 25-OH-Vitamin-D₃.

Abweichend zu Beispiel 3 wurden die Messproben durch Extraktion (in Einzelwerten) aufgereinigt. Hierzu wurde jeweils 50 µl Probe [nicht-spezifischer Assaypuffer NSB, Standard, Kontrolle, Patientenprobe (Plasma, Serum oder Urin)] in ein 1.5 ml Einmalreaktionsgefäß gegeben, 200 µl Acetonitril hinzugefügt, gemischt, die GefäBwände freizentrifugiert, und die Mischung 20 bis 30 Minuten bei 4°C inkubiert. Das Gemisch wurde 10 Minuten bei 1700 x g zentrifugiert. Die Bestimmungen erfolgten in Doppelwerten mit den Überständen.

Hierzu wurde 25 µl klarer Überstand in ein Glasröhrchen (oder in ein Spezial-RIA-Gefäß von Sarstedt, Darmstadt) überführt, und 10 µl Tracer (³H-25-OH-D), 300 µl Assaypuffer und 100 µl Vitamin-D-bindendes Protein (nicht in NSB) hinzugeben. Der Röhrcheninhalt wurde gemischt, eine Stunde bei 4°C inkubiert. und zum Entfernen von nicht gebundenem radioaktiven Tracer 100 µl Aktivkohlesuspension (aktivkohlehaltiger Phosphatpuffer mit 0,1% NaN₃) zugegeben. Der Röhrcheninhalt wurde gemischt, 3 bis 5 Minuten bei 4°C inkubiert, und die Aktivkohle durch 10 Minuten Zentrifugieren bei 1700 x g pellettiert. Es wurde dann jeweils 400 µl Überstand in ein Zählgefäß (7 ml) überführt und nach Zugabe von 2 ml Szintillatorflüssigkeit wie Aquasafe™ 300 oder HiSafe™ III die im Überstand befindliche Radioaktivität gezählt (2 Minuten in einem Beta-Counter). Die Messwerte für die Kontrollen nach Erstellung der Eichkurve sind in Tabelle 5 gezeigt.

Der Vergleich mit dem ELISA nach Beispiel 3 zeigt, dass für beide Assayverfahren (ELISA und CBPA) gilt, dass der Normbereich für 25-OH-Vitamin-D im Plasma oder Serum etwa 25 - 125 nmol/l ist. Die Nachweisegrenze dieser Testsysteme wurde festgesetzt als B₀ + 2SD. Sie beträgt etwa 2,5 nmol/l.

Kreuzreaktionen: Zu Aktivkohle behandeltem Serum wurde 25-OH-Vitamin-D₂ (125 nmol/l), 24,25-(OH)₂-Vitamin-D₃ (250 nmol/l) und 1,25-(OH)₂-Vitamin-D₃ (250 nmol/l) zugesetzt. Das 25-OH-Vitamin-D₂ reagierte zu 60%, das 24,25-(OH)₂-Vitamin-D₃ zu 100% kreuz, während das 1,25-(OH)₂-Vitamin-D₃ keine Kreuzreaktivität zeigte. Ähnliche Ergebnisse werden auch für erfindungsgemäße multifunktionelle 25-OH-Vitamin-D-Konjugate gefunden bzw. erwartet.

Reproduzierbarkeit: In Wiederholungsmessungen (n=11) einer 25-Dihydroxy-Vitamin-D₃haltigen Probe wurden nachstehende Resultate erzielt. Ähnliches gilt auch für Messungen mit Hilfe der multifunktionellen 25-OH-Vitamin-D-Konjugate gemäß der Erfindung:

**Tabelle IV**

| Intraassayvarianz: | | | |
|---|---|---|---|
| | Anzahl | Mittelwert nmol/l | VK % |
| Probe 1 | 32 | 11.3 | 12.5 |
| Probe 2 | 32 | 318 | 7.2 |

| Interassayvarianz | | | |
|---|---|---|---|
| | Anzahl | Mittelwert nmol/l | VK % |
| Probe 1 | 9 | 9,9 | 17 |
| Probe 2 | 9 | 310 | 11 |

| Klinik: | | | |
|---|---|---|---|
| | Anzahl | Mittelwert nmol/l | |
| Normalpersonen | 35 | 54 | |
| Patienten mit Schenkelhalsbrüchen | 43 | 9,5 | |

Für die in Beispiel 3 genannten Proben wurden mit den Verfahren nach Beispiel 3 und 4 folgende 25-OH-Vitamin-D-Konzentrationen ermittelt.

**Tabelle V**

| Serumproben Nr. | ELISA mit 25-OH-D-Biotin nMol/L | CBPA mit ³H-25-OH-D nMol/L | Kontrollen | ELISA mit 25-OH-D-Biotin nMol/L | Alternative Bestimmung nMol/L |
|---|---|---|---|---|---|
| 24 | 32.9 | 33.3 | K1 | Fehlwert | 20 (a) |
| 203 | 36.8 | 29.19 | K2 | 76.8 | 75-125 (a) |
| 963 | 48.9 | 38.4 | K3 | 15.0 | 20-33 (b) |
| 965 | 21.8 | 15.8 | K4 | 51.3 | 72-120 (b) |
| NP 18 | 57.0 | | | | |
| NP 25 | 67.9 | | | | |
| NP 34 | 82.4 | | | | |
| NP 37 | 72.9 | | | | |

| | | | | | |
|---|---|---|---|---|---|
| (a) CBPA mit ³H-25-OH-D | | | | | |
| (b) Herstellerangabe | | | | | |

Die von den Herstellern angegeben Werte waren generell höher als die im kompetitiven Bindungsassay ermittelten Konzentrationen. Dies lässt vermuten, dass sich in den überlassenen Proben bereits ein erheblicher Teile des 25-OH-Vitamin-D zersetzt bzw. durch Lichteinwirkung umgelagert hatte.

### Beispiel 5: Kontrolle der ELISA-Bestimmung durch HLPC

Es wurden daher in verschiedenen Proben die 25-OH-Vitamin-D-Konzentration durch ELISA gemäß Beispiel 3 und zur Kontrolle durch HPLC nachbestimmt. Für die Eichkurve wurden Standards verwendet mit Vitamin-D₃-Konzentrationen von 0, 8, 20, 50, 125 und 312 nMol/l. Alle Proben und Standards wurden in Doppelwerten bestimmt. Die 25-OH-Vitamin-D₃-Konzentration der Proben wurde dann anhand der Eichkurve aus den Mittel der Doppelwerte berechnet.

Die Ergebnisse sind in nachstehender Tabelle VI gezeigt.

**Tabelle VI**

| Probe | 25-OH-Vitamin-D₃ [nMol/L] | |
|---|---|---|
| | HPLC | ELISA |
| 1 | 20-33 | 30 |
| 2 | 72-120 | 76 |
| 3 | 79-102 | 96 |
| 4 | < 15 | < Nachweisgrenze |
| 5 | <15 | 7.4 |

### Beispiel 6: Langzeitstabilität von 25-Hydroxyvitamin-D-Konjugat in der ELISA-Bestimmung.

Es wurden Eichkurven mit gleichen Standardlösungen und Reagenzien gemäß dem Beispiel 3 nach 60 und 100 Tagen wiederholt, um zu ermitteln, inwieweit sich eine ELISA-Bestimmung mit erfindungsgemäßen Biotin-25-OH-Vitamin-D-Konjugat im Laufe der Zeit verändert, wenn die Reagenzien zwischenzeitlich bei 4 bis 6°C im Dunkeln gelagert werden. Die nachstehende Tabelle zeigt die jeweiligen optischen Dichten nach 30 Minuten Entwicklung (siehe Beispiel 3)

**Tabelle VII**

| **Standard** | Standard | Doppelwert zu Spalte 1 | Nach 60 Tagen | Doppelwert zu Spalte 3 | Nach 100 Tagen | Doppelwert zu Spalte 5 |
|---|---|---|---|---|---|---|
| nMol/L | **1** | **2** | **3** | **4** | **5** | **q6** |
| **NSB** | --- | --- | 0,191 | 0,280 | 0.088 | 0,109 |
| **0** | 2.256 | 2.182 | 2.227 | 2.285 | 1,471 | 1.562 |
| **8** | 1,845 | 1,861 | 2,041 | 2.125 | 1,345 | 1.366 |
| **20** | 1.432 | 1,456 | 1.860 | 1.903 | 1.079 | 1.060 |
| **50** | 0,625 | 0.612 | 1.293 | 1.214 | 0,610 | 0,690 |
| **125** | 0,287 | 0,261 | 0.606 | 0.615 | 0,442 | 0.329 |
| **312** | 0.156 | 0.176 | 0.448 | 0.434 | 0.293 | 0.237 |

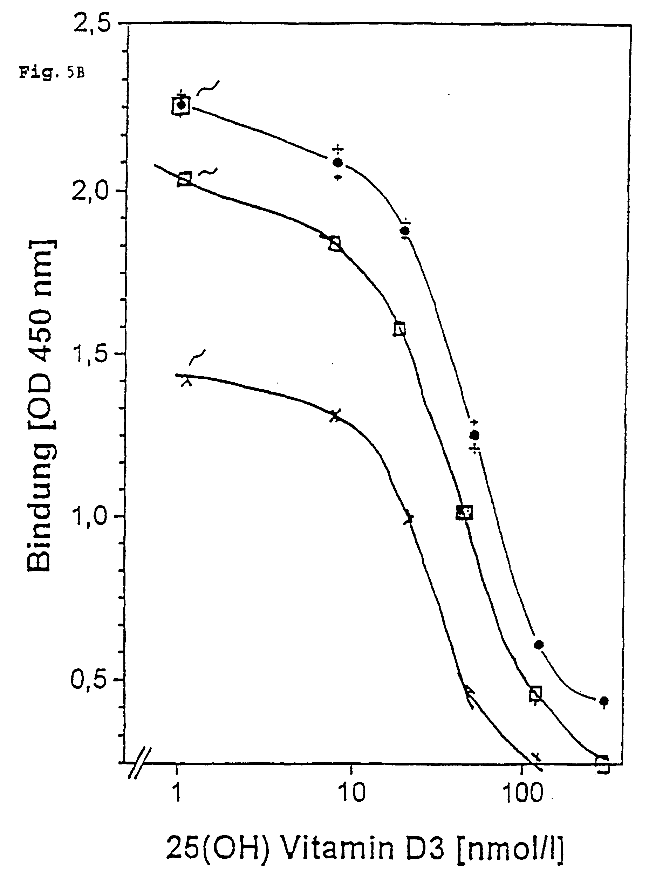

Werden die Werte der verschiedenen Eichkurven, abzüglich der jeweiligen nichtspezifischen Bindung, in ein Diagramm aufgetragen (siehe **Fig. 5B).** so ist leicht ersichtlich, dass die Eichkurven abgesehen einer relativen, vertikalen Verschiebung die gleiche Form besitzen. Dies zeigt, dass sich die Empfindlichkeit und Spezifität der ELISA-Bestimmungen über den genannten Zeitraum nicht verändert hat.

### Beispiel 7: 25(OH)-Vitamin-D₃-ELISA-MTP mit anti-Vitamin-D-Bindungsprotein

Der Versuch erfolgte im Wesentlichen nach dem Protokoll von Beispiel 3 und dem in **Fig. 4** gezeigten Prinzip. Es wurden folgende Puffer verwendet: a) Waschpuffer. PBS, pH 7.4 mit 0.05% Tween-20; b) Assaypuffer: 5 g Casein wurden in 100 ml 0.1 N NaOH gelöst und mit PBS, pH 7,4 auf 1 l aufgefüllt. Dann wurden 3% (w/v) PEG-6000 sowie 0.1 g Thimerosal™ hinzugefügt. Alle Inkubationen erfolgten im Dunkeln und unter Schütteln.

### (i) Beschichtung der Mikrotiterplane

In die Vertiefungen einer Mikrotiterplatte wurden jeweils 100 µl Kaninchen-anti-Vitamin-D-Bindungsprotein in 60 mM NaHCO₃, pH 9,6 gegeben und die Platte über Nacht bei 4°C inkubiert. Die Lösungen wurden entfernt und jede Vertiefung fünfmal mit 200 µl Waschpuffer gewaschen. Dann wurde 250 µl Assaypuffer in jede Vertiefung gegeben und die Platte eine Stunde bei Raumtemperatur inkubiert. Der Assaypuffer wurde entfernt und eine jede Vertiefung fünfmal mit je 200 µl Waschpuffer gewaschen.

### (ii) Probenvorbereitung

Es wurde 50 µl Serum, Plasma oder Standard in einem 1.5 ml Eppendorf-Reaktionsgefäß mit 200 µl Ethanol_{abs} (vorgekühlt auf -20 C) gemischt, gevortext und dann 20 Minuten bei -20°C ausgefällt. Die Proben wurden in einer Eppendorf-Tischzentrifuge bei maximaler Umdrehung zentrifugiert. Der Überstand wurde abgenommen und im ELISA eingesetzt.

### (iii) ELISA

Als Erstes wurde in eine jede Vertiefung 100 µl Vitamin-D-Bindungsprotein, verdünnt in Assaypuffer, gegeben und eine Stunde bei Raumtemperatur inkubiert. Dann wurde die Platte ausgeschlagen und eine jede Vertiefung fünfmal mit je 200 µl Waschpuffer gewaschen.

Danach wurden je 100 µl Biotin-Vitamin-D, verdünnt in Assaypuffer, zusammen mit 10µl Standard, Probe oder Kontrolle in die Vertiefungen gegeben. Die Platte wurde 24 Stunden bei 4°C inkubiert. Die Lösungen wurden wiederum entfernt und eine jede Vertiefung fünfmal mit je 200 µl Waschpuffer gewaschen.

Als Drittes wurde jeweils 100 µl Peroxidase-gekopppeltes Streptavidin einer 1:10000-Verdünnung in Waschpuffer in die Vertiefungen gegeben und 45 Minuten bei Raumtemperatur inkubiert. Die Platte wurde ausgeschlagen und jede Vertiefung fünfmal mit je 200 µl Waschpuffer gewaschen.

Für die Farbreaktion wurden 100 µl TMB-Substrat-Lösung in jede Vertiefung gegeben. Nach ausreichender Farbentwicklung (30 Minuten) wurde die Reaktion mit 50 µl 2M H₂SO₄ pro Vertiefung beendet. Die Messungen der optischen Dichte erfolgte bei 450 nm. Es wurden ähnliche bis gleiche Ergebnisse erhalten, wie in Beispiel 3 bzw. Tabelle V.

### Beispiel 8: Inhalt einer Testpackung bzw. eines Reagenziensatzes zur Bestimmung von 25-Hydroxyvitamin-D und 1α,25-Dihydroxyvitamin-D:

Inhalt der Testpackung bzw. Testreagenzien und deren Vorbereitung:
**Standards**, bspw. 6 Fläschchen 25-OH-Vitamin-D-Standards mit den Konzentrationen 0, 8, 20, 50, 125 und 312 nmol/l; gebrauchsfertig in Waschpuffer.
**Mikrotiterplatten**, bspw. mit Streptavidin beschichtet, steril verpackt und vorgewaschen.
**Pufferlösungen**, bspw. Waschpuffer, NSB-Puffer und Assaypuffer, Stopplösung.
**Kontrollen**, bspw. zwei Fläschchen 25-OH-Vitamin-D-Kontrollen in Humanserum. Kontrolle 1 (30 nMol 25-OH-D/L), Kontrolle 2 (80 nMol 25-OH-D/L).
**Tracer**, bspw. ein Fläschchen mit Biotin-Vitamin-D (25-OH-Vitamin-D₃-3β-3'[6-N-(biotinyl)-hexamido]amidopropylether) in Waschpuffer (100 ng/ml)
**Vitamin-D-Bindungsprotein**, bspw. ein Fläschchen mit Bindungsprotein aus Ziegenserum in Phosphatpuffer mit 0,1 % NaN₃ als Stabilisator.
**Marker**, bspw. ein Fläschchen anti-Kaninchen-lgG-Peroxidase in Waschpuffer.
**TMB-Entwicklerlösung**, bspw. ein Fläschchen stabilisierte Tetramethylbenzidin-Entwicklerlösung in Waschpuffer.

### Beispiel 9: ELISA zur quantitativen Bestimmung von 1,25-Dihydroxyvitamin-D

Die Bestimmung von 1,25-Vitamin-D₃ erfolgte nach dem in **Fig. 2** dargestellten Prinzip, nur dass als Tracer 1,25-Dihydroxy-Vitamin-D₃-Biotin-Verbindung diente. Bei der Kompetition wird 1,25-Dihydroxyvitamin-D₃ aus Standard oder Probe zusammen mit einem 1,25-Dihydroxy-Vitamin-D-Bindungsprotein, einem monoklonalen Maus-anti-1α,25-Dihydroxyvitamin-D-Antikörper (B. Mawer et al. in Steroids, 1985, 46, 741-754), zusammengegeben. Das 1,25-Dihydroxyvitamin-D; aus Standard oder Probe und die immobilisierte 1,25-Dihydroxyvitamin-D₃-Biotin-Verbindung konkurrieren dann um die Bindungsstelle des Antikörpers. Der Nachweis erfolgt mittels Peroxidase-markierten Antikörpern (Ziege-anti-Maus-IgG-POX).
(i) Die Beschichtung der Mikrotiterplatten mit Streptavidin erfolgte wie in Beispiel 3, wobei aber der Waschpuffer 0.1% Triton™ X-100 als Detergens enthielt. Anders als in Beispiel 3 wurden die Vertiefungen der Mikrotiterplatte nach der Behandlung mit der Streptavidin-Lösung nicht mehr mit Waschpuffer gewaschen, sondern jeweils eine Stunde mit 250 µl wässriger Sorbitol-Lösung (Karion™ F 1:4 in Wasser) behandelt. Die Bindung des Tracers (1,25-Dihydroxyvitamin-D-Biotin) erfolgte wie in Beispiel 3, nur dass in jede Vertiefung 200 µl Tracer-Lösung gegeben wurde (20 ng 1,25-Dihydroxy-Vitamin-D-3β-3'[6-N-(biotinyl)hexamido]amidopropylether in Waschpuffer). Das 1,25-Dihydroxyvitamin-D-Biotin wurde wie in **Fig. 1** schematisch aufgezeigt synthetisiert, nur dass nach dem ersten Schritt die im Unterschuss 3-cyanoethylierte 1-OH-Vitamin-D-Zwischenverbindung isoliert wurde. Es kann aber auch beliebig eine der nachfolgenden Zwischenverbindungen bzw. nach einer Mischsynthese spezifisch der 1,25-Dihydroxy-Vitamin-D-3β-3'[6-N-(biotinyl)hexamido]amidopropylether mittels HPLC isoliert werden.
(ii) Da im menschlichen Serum das Verhältnis von 25-OH-Vitamin-D₃ zu 1,25-Dihydroxyvitamin-D₃ in der Regel im Bereich von 1000:1 liegt, bedarf die quantitative Bestimmung von 1,25-Dihydroxyvitamin-D einer gründlichen Vorbereitung der Proben durch eine kombinierte Verteilungs- und Adsorptionschromatographie. Im ersten Schritt wurden hierzu Extrelut™-Kieselgur-Säulen (Merck, Darmstadt) mit je 500 µl Tris-Puffer äquilibriert und dann auf die Säulen jeweils 500 µl Standard, Kontrolle oder Untersuchungsprobe - in Duplikaten - aufgetragen; die Proben konnten dann 10 Minuten auf den Säulen einziehen. Die Abtrennung der Vitamin-D-Verbindungen von der Extrelut™-Säule erfolgte durch 4 mal 1 ml Diisopropylether im Abstand von jeweils drei Minuten. Der Extrelut™-Extrakt wurde unmittelbar auf eine Silica-Kartusche (Merck, Darmstadt) überführt und die Extrelut™-Säule verworfen. Die Silicasäule wurde 5 mal mit 2 ml Isopropanol/Hexan. (4/96 v/v) und 3 mal mit 2 ml Isopropanol/Hexan (6/94 (v/v)) gewaschen. Das 1,25-Dihydroxy-Vitamin-D wurde dann mit 2 mal 2 ml Isopropanol/Hexan (25/75 v/v) von der Silicasäule eluiert und unter Stickstoffstrom bei 37°C oder in einer Vakuumzentrifuge getrocknet. Die Standard- und Untersuchungsproben wurden schließlich in 20 µl Ethanol p.a. aufgenommen, mit je 200 µl Maus-anti-1,25-Dihydroxyvitamin-D-Antikörperlösung (1: 150000 in RRA-Assaypuffer: 50 mM KH₂PO₄, 15 mM KCI, 1,25 mM EDTA, 3mM Mercaptoethanol, pH 7,5) versetzt und eine Stunde bei Raumtemperatur vorinkubiert- möglichst zeitgleich mit der Belegung der Streptavidin-behandelten Mikrotiterplatte mit dem 1,25-Dihydroxyvitamin-D-Biotin-Tracer.
(iii) Die Vertiefungen der Tracer-beschichteten Mikrotiterplatte wurden 5 mal mit je 300 µl Triton™-Waschpuffer gewaschen und auf saugfähigem Papier ausgeschlagen. Dann wurde 200 µl Antikörper-Probenlösung aus der Vorinkubation in die Vertiefungen überführt und eine Stunde im Dunkeln und unter Schütteln bei Raumtemperatur inkubiert. Nach dem Entfernen der Lösungen aus den Vertiefungen wurden diese fünfinal mit je 200 µl Waschpuffer gewaschen. Die quantitative Bestimmung erfolgte analog Beispiel 3 durch eine Stunde Inkubation mit 200 µl Kaninchen-anti-Maus-IgG-Peroxidase (1:10000 in Waschpuffer) bei Raumtemperatur, fünfmal Waschen der Vertiefungen mit 300 µl Waschpuffer, eine Farbreaktion im Dunkeln mit 200 µl TMB-Substratlösung (gebrauchsfertig von NOVUM Diagnostika GmbH, Dietzenbach) , Stoppen der Farbreaktion nach 15 Minuten durch Zugabe von 50 µl 2 M H₂SO₄ und Bestimmen der Extinktion bei 450 nm.

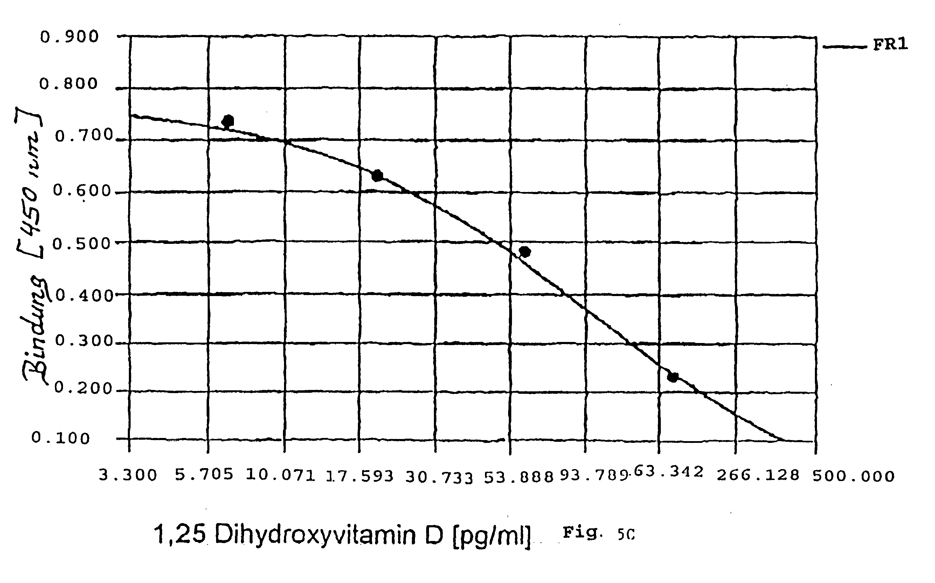

Die nachstehende Tabelle VIII zeigt die Ergebnisse der 1,25-Dihydroxyvitamin-D-Bestimmung im Serum von 11 Dialysepatienten und sechs willkürlich ausgewählten Normalpersonen. Zur Ermittlung der Eichkurve bzw. als Standard wurden Lösungen von 1,25-Dihydroxyvitamin-D in Assaypuffer mit folgenden Konzentrationen eingesetzt: 0, 6,6, 20, 60 und 180 pg/ml (siehe Eichkurve in **Fig. 5C**).

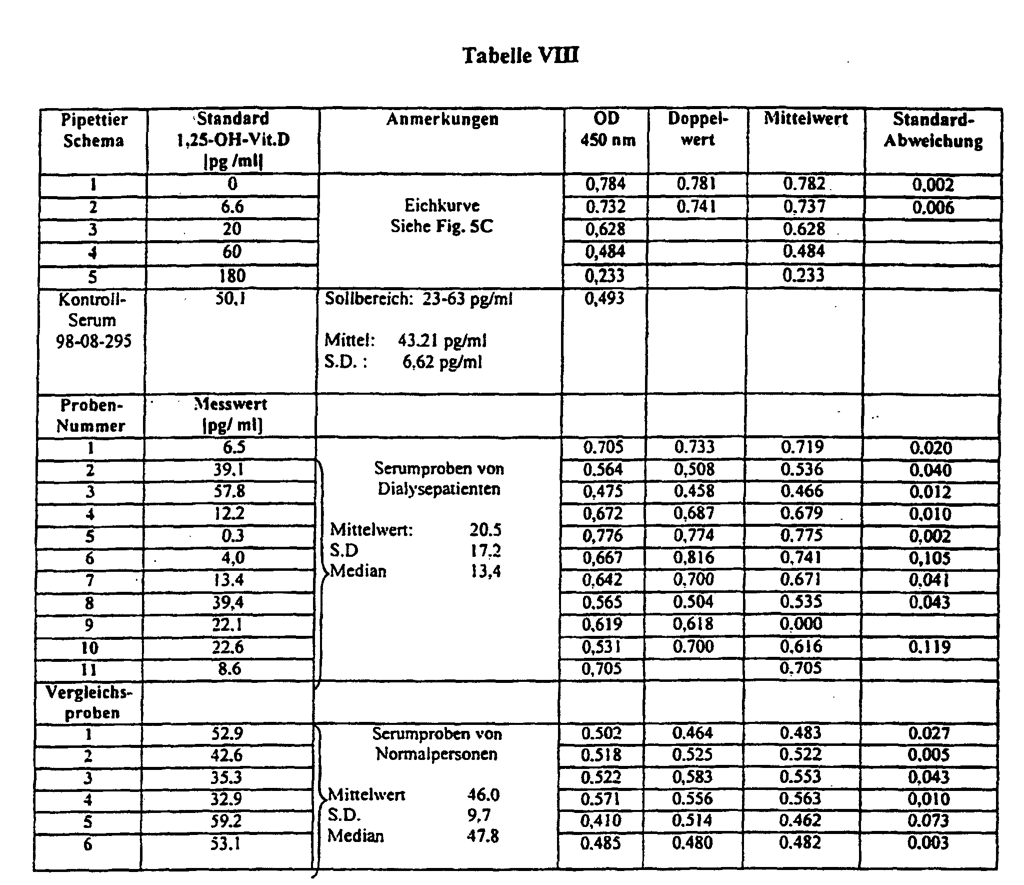

**Fig. 14** veranschaulicht in einem Balkendiagramm nochmals die gefundenen Werte für Dialyse- und Normalpatienten, wonach das Serum von Dialysepatienten im Schnitt deutlich weniger aktives 1,25-Dihydroxyvitamin-D enthält. Die große Varianz der Werte bei den Dialysepatienten zeigt zudem die Notwendigkeit, bei Dialysepatienten den Gehalt an aktiviertem 1,25-Dihydroxyvitamin-D im Serum noch verstärkt zu kontrollieren, um den typischen Folgen eines Vitamin-D-Mangels besser entgegentreten zu können.

## Patentansprüche

1. Verfahren zur Herstellung eines Vitamin-D-Derivats der Formel: 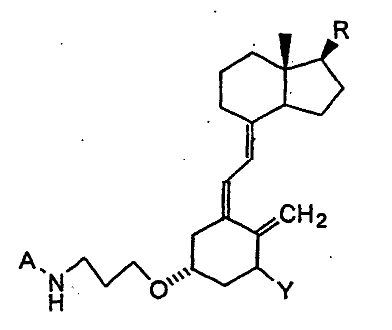 worin ist:
R eine 25-Hydroxy-Seitenkette von Vitamin-D₂ beziehungsweise Vitamin-D₃;
Y Wasserstoff oder Hydroxy;
A eine über eine Abstandsgruppe gebundene funktionelle Gruppe, die von einem Protein mit hoher Affinität gebunden werden kann;
**gekennzeichnet durch** die Schritte:
a) Cyanoethylierung der 3-Hydroxy-Gruppe einer Vitamin-D-Ausgangsverbindung in einem geeigneten Lösungsmittel wie Acetonitril in Gegenwart von Kaliumhydrid und *tert*-Butanol;
b) Zugabe von Lithiumhydrid und Überführen der 25-Hydroxy-Gruppe in das Lithiumalkoholat und anschließend Reduktion der Nitrilgruppe mit Lithiumaluminiumhydrid; und
c) Anhãngen einer Abstandsgruppe zusammen mit einer funktionellen Gruppe A an die Aminopropylether-Seitenkette.

2. Verfahren nach Anspruch 1, wobei die funktionelle Gruppe A ausgewählt wird aus Biotin, Digoxigenin, Tyrosin, FITC-substituiertem Tyrosin, substituierten Aminosäuren, charakteristischen Aminosäure- und Peptidsequenzen, FITC, Proteinen und Peptidgruppen, Protein-A, Protein-G und Vitamin-D-Derivaten.

3. Verfahren nach Anspruch 1, wobei die funktionelle Gruppe A 25-Hydroxy-Vitamin-D oder 1α,25-Dihydroxy-Vitamin-D ist.

4. Verfahren nach Anspruch 3, wobei die funktionelle Vitamin-D-Gruppe in 3β-Stellung über eine Etherbrücke mit der Abstandsgruppe gekoppelt ist.

5. Verfahren nach Anspruch 2, wobei der Schritt c) erfolgt mit Biotinyl-N-ε-aminocaproyl-hydroxy-succinimidester (LC-BHNS) oder einem aktivierten Biotinylierungsreagens.

6. Verfahren nach Anspruch 1, wobei die Abstandsgruppe eine Aminocarbonsäure-, ein Aminoundecansäure- oder ein Aminopolyetherrest ist.

7. Verfahren zur Herstellung der 3-Aminopropyl-25-Hydroxy- oder 3-Aminopropyl-1α,25-dihydroxy-Vitamin-D-Zwischenverbindung, **gekennzeichnet durch** die Verfahrenschritte a) und b) nach Anspruch 1.

8. Verfahren zur quantitativen Bestimmung von 25-Hydroxy- und 1α,25-Dihydroxy-Vitamin-D-Metabolit in einer Probe, **dadurch gekennzeichnet, dass** ein Vitamin-D-Derivat mit einem Verfahren nach einem der Ansprüche 1 bis 6 erhalten und als Bindungspartner eingesetzt wird.

9. Verfahren nach Anspruch 8, wobei das Verfahren ein kompetitiver Immunoassay ist.

10. Verfahren nach Anspruch 8, wobei das Verfahren ein Sandwich-Immunoassay ist.

11. Reagenziensatz zur Bestimmung von 25-Hydroxy- und 1α,25-Dihydroxy-Vitamin-D-Metaboliten nach einem Verfahren gemäß irgendeinem Anspruch 8 bis 10, **dadurch gekennzeichnet, dass** er eine standardisierte Menge Feststoff oder Lösung eines Vitamin-D-Derivats enthält, das gemäß einem der Ansprüche 1 bis 6 hergestellt ist.

## Claims

1. A method of producing a vitamin D derivative of the formula: 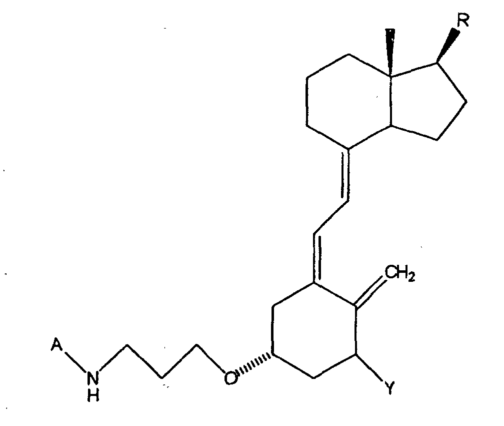 wherein:
R represents a 25-hydroxy side-group of vitamin D₂ or of vitamin D₃, respectively;
Y represents hydrogen or hydroxy;
A represents a functional group, coupled via a spacer group, which can be bound by a protein with high affinity;
**characterised by** the steps:
a) cyanoethylation of the 3-hydroxy group of a vitamin D starting compound in a suitable solvent such as acetonitrile in the presence of potassium hydride and tertiary butanol;
b) addition of lithium hydride and conversion of the 25-hydroxy group into the lithium alcoholate and subsequent reduction of the nitrile group with lithium aluminium hydride; and
c) linking a spacer group together with a functional group A on the amino propylether side chain.

2. Method according to claim 1, wherein the functional group A is selected from biotin, digoxigenin, tyrosine, FITC substituted tyrosine, substituted amino acids, characteristic amino acid- and peptide sequences, FITC, proteins and peptide groups, protein-A, protein-G and vitamin D derivates.

3. Method according to claim 1, wherein the functional group A is 25-hydroxy vitamin D or 1α,25-dihydroxy vitamin D.

4. Method according to claim 3, wherein the functional vitamin D group is coupled in 3β-position via an ether bridge with the spacer group.

5. Method according to claim 2, wherein step c) is effected with biotinyl-N-ε-amino caproyl-hydroxy-succinimide ester (LC-BHNS) or an activated biotinylation reagent.

6. Method according to claim 1, wherein the spacer group is an amino carboxylic acid residue, an amino undecanoic acid residue or an amino polyether residue.

7. Method of producing the 3-amino propylether-25-hydroxy- or 3-amino propylether-1α, 25-dihydroxy vitamin D intermediate, **characterised by** the method steps a) and b) according to claim 1.

8. Method for the quantitative determination of 25-hydroxy- and 1α,25-dihydroxy vitamin D metabolite in a sample, **characterised in that** a vitamin D derivative is obtained with a method according to any one of claims 1 to 6, and is employed as a binding partner.

9. Method according to claim 8, wherein the method is a competitive immunoassay.

10. Method according to claim 8, wherein the method is a sandwich immunoassay.

11. Reagent kit for the determination of 25-hydroxy- and 1α,25-dihydroxy vitamin D metabolites according to a method as defined in any one of claims 8 to 10, **characterised in that** it contains a standardized quantity of solid or solution of a vitamin D-derivative which is obtained in accordance with any one of claims 1 to 6.

## Revendications

1. Procédé pour fabriquer un dérivé de la vitamine D selon la formule : 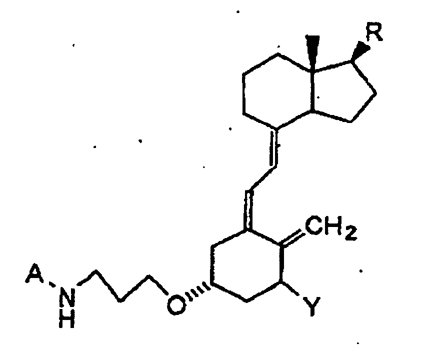 dans laquelle :
R est une chaîne latérale de 25 - hydroxy vitamine D2, notamment de la vitamine D3;
Y de l'hydrogène ou de l'hydroxy
A un groupe fonctionnel lié au moyen d'un groupe allongé qui peut être relié à une protéine à affinité élevée; **caractérisé par** les étapes suivantes :
a) cyanoéthylation du groupe 3-hydroxy d'une liaison de départ de vitamine D dans un solvant approprié comme de l' acétonitrile en présence de hydrure de potassium et de l'alcool butylique tertiaire ;
b) l'ajout de hydrure de lithium et transformation du groupe 25-hydroxy dans l'alcoolate de lithium puis réduction du groupe nitrile avec de l'hydrure de lithium-aluminium ; et
c) accrochage d'un groupe allongé ensemble avec un groupe fonctionnel A à la chaîne latérale d'éther d'aminopropyle.

2. Procédé selon la revendication 1 où le groupe fonctionnel A est choisi parmi la biotine , la dioxygénine, la tyrosine, la tyrosine FITC substituée , les acides aminés substitués , les acides aminés et les séquences de peptides caractéristiques, la FITC, les protéines et les groupes de peptides , la protéine A, la protéine G et les dérivés de la vitamine D.

3. Procédé selon la revendication 1, où le groupe fonctionnel A est de la 25-hydroxy vitamine D ou de la 1α, 25 dihydroxy - vitamine D.

4. Procédé selon la revendication 3, où le groupe fonctionnel de vitamine D est couplé dans la position 3β par un pont d'éther avec le groupe allongé.

5. Procédé selon la revendication 2, où l'étape c) est effectué avec biotinyle -N-ε-aminocaproyl-hydroxy-succin-imidester (LC-BHNS) ou un agent de biotinylation activé.

6. Procédé selon la revendication 1, où le groupe allongé est un acide d'aminocarbone, un reste d'acide d'aminoundécane ou d'aminopolyéther.

7. Procédé pour fabriquer la liaison intermédiaire 3-Aminopropyl-25-Hydroxy ou 3-Aminopropyl-1α, 25-dihydroxy-vitamine-D, **caractérisé par** les étapes du procédé a) et b) selon la revendication 1.

8. Procédé pour déterminer de façon quantitative du 25-Hydroxy et 1α, 25-Dihydroxy-Vitamine-D-metabolite dans un échantillon, **caractérisé en ce qu'**un dérivé de vitamine D est obtenu selon une des revendications 1 à 6 et est employé en tant que partenaire de liaison.

9. Procédé selon la revendication 8, où le procédé est un immunoessai compétitif.

10. Procédé selon la revendication 8, où le procédé est un sandwich d'immunoessai.

11. Un jeux d'agents pour déterminer les 25-Hydroxy et 1α, 25-Dihydroxy-Vitamine-D-metabolites selon un procédé conforme à n'importe laquelle des revendications 8 à 10, **caractérisé en ce qu'**il comprend une quantité standard de matières solides ou une solution d'un dérivé de vitamine D, qui est fabriqué selon une des revendications 1 à 6.
